# EUROPEAN PATENT APPLICATION

(11) **EP 3 757 125 A1**
(43) Date of publication of application: **30.12.2020**
(21) Application number: 19183405.0
(22) Date of filing: 28.06.2019
(51) Int. Cl.: C07K 16/18, A61P 25/28

(54) **ANTIBODY DIRECTED AGAINST THE APOE AMINO-TERMINAL FRAGMENT OF 12 KDA**

(71) Applicant: BioArctic AB, 112 51 Stockholm (SE)
(72) Inventor: SAHLIN, Charlotte, 112 51 Stockholm (SE); FÄLTING, Johanna, 112 51 Stockholm (SE); ERIKSSON, Maria, 112 51 Stockholm (SE); MÖLLER, Christer, 112 51 Stockholm (SE)
(74) Representative: Boult Wade Tennant LLP

(57) **Abstract**

The disclosure relates to an antibody or antigen binding portion thereof, which binds to a neo-epitope of a C-terminal fragment of apolipoprotein E, to methods of producing such an antibody or antigen binding portion thereof, and to therapeutic and diagnostic uses thereof.

SEQUENCE LISTING

<110>  BioArctic AB

<120>  NEW ANTIBODY

<130>  NLW/P207695EP00

<160>  55

<170>  PatentIn version 3.5

<210>  1
<211>  100
<212>  PRT
<213>  Artificial

<220>
<223>  Synthetic peptide

<400>  1

Gly Gln Pro Leu Gln Glu Arg Ala Gln Ala Trp Gly Glu Arg Leu Arg
1               5                   10                  15

Ala Arg Met Glu Glu Met Gly Ser Arg Thr Arg Asp Arg Leu Asp Glu
            20                  25                  30

Val Lys Glu Gln Val Ala Glu Val Arg Ala Lys Leu Glu Glu Gln Ala
        35                  40                  45

Gln Gln Ile Arg Leu Gln Ala Glu Ala Phe Gln Ala Arg Leu Lys Ser
    50                  55                  60

Trp Phe Glu Pro Leu Val Glu Asp Met Gln Arg Gln Trp Ala Gly Leu
65                  70                  75                  80

Val Glu Lys Val Gln Ala Ala Val Gly Thr Ser Ala Ala Pro Val Pro
                85                  90                  95

Ser Asp Asn His
            100

<210>  2
<211>  101
<212>  PRT
<213>  Artificial

<220>
<223>  Synthetic peptide

<400>  2

Ala Gly Gln Pro Leu Gln Glu Arg Ala Gln Ala Trp Gly Glu Arg Leu
1               5                   10                  15

Arg Ala Arg Met Glu Glu Met Gly Ser Arg Thr Arg Asp Arg Leu Asp
            20                  25                  30

Glu Val Lys Glu Gln Val Ala Glu Val Arg Ala Lys Leu Glu Glu Gln
        35                  40                  45

Ala Gln Gln Ile Arg Leu Gln Ala Glu Ala Phe Gln Ala Arg Leu Lys
    50                  55                  60

Ser Trp Phe Glu Pro Leu Val Glu Asp Met Gln Arg Gln Trp Ala Gly
65                  70                  75                  80

Leu Val Glu Lys Val Gln Ala Ala Val Gly Thr Ser Ala Ala Pro Val
                85                  90                  95

Pro Ser Asp Asn His
            100

<210>  3
<211>  102
<212>  PRT
<213>  Artificial

<220>
<223>  Synthetic peptide

<400>  3

Leu Ala Gly Gln Pro Leu Gln Glu Arg Ala Gln Ala Trp Gly Glu Arg
1               5                   10                  15

Leu Arg Ala Arg Met Glu Glu Met Gly Ser Arg Thr Arg Asp Arg Leu
            20                  25                  30

Asp Glu Val Lys Glu Gln Val Ala Glu Val Arg Ala Lys Leu Glu Glu
        35                  40                  45

Gln Ala Gln Gln Ile Arg Leu Gln Ala Glu Ala Phe Gln Ala Arg Leu
    50                  55                  60

Lys Ser Trp Phe Glu Pro Leu Val Glu Asp Met Gln Arg Gln Trp Ala
65                  70                  75                  80

Gly Leu Val Glu Lys Val Gln Ala Ala Val Gly Thr Ser Ala Ala Pro
                85                  90                  95

Val Pro Ser Asp Asn His
            100

<210>  4
<211>  6
<212>  PRT
<213>  Artificial

<220>
<223>  Synthetic peptide

<400>  4

Leu Ala Gly Gln Pro Leu
1               5


<210>  5
<211>  6
<212>  PRT
<213>  Artificial

<220>
<223>  Synthetic peptide

<400>  5

Ala Gly Gln Pro Leu Gln
1               5


<210>  6
<211>  6
<212>  PRT
<213>  Artificial

<220>
<223>  Synthetic peptide

<400>  6

Gly Gln Pro Leu Gln Glu
1               5


<210>  7
<211>  7
<212>  PRT
<213>  Artificial

<220>
<223>  Synthetic peptide

<400>  7

Leu Ala Gly Gln Pro Leu Gln
1               5


<210>  8
<211>  7
<212>  PRT
<213>  Artificial

<220>
<223>  Synthetic peptide

<400>  8

Ala Gly Gln Pro Leu Gln Glu
1               5


<210>  9
<211>  8
<212>  PRT
<213>  Artificial

<220>
<223>  Synthetic peptide

<400>  9

Leu Ala Gly Gln Pro Leu Gln Glu
1               5


<210>  10
<211>  5
<212>  PRT
<213>  Artificial

<220>
<223>  Synthetic peptide

<400>  10

Ser Tyr Ala Met Ser
1               5


<210>  11
<211>  17
<212>  PRT
<213>  Artificial

<220>
<223>  Synthetic peptide

<400>  11

Glu Ile Ser Gly Ser Gly Ser Arg Asp His Tyr Thr Asp Ser Val Thr
1               5                   10                  15


Gly



<210>  12
<211>  12
<212>  PRT
<213>  Artificial

<220>
<223>  Synthetic peptide

<400>  12

Gln Leu Thr Gly Thr Asp Tyr Tyr Gly Thr Asp Tyr
1               5                   10

<210>  13
<211>  17
<212>  PRT
<213>  Artificial

<220>
<223>  Synthetic peptide

<400>  13

Glu Ile Ser Ser Gly Gly Gly Ser Thr Asn Tyr Leu Asp Thr Val Thr
1               5                   10                  15

Gly

<210>  14
<211>  12
<212>  PRT
<213>  Artificial

<220>
<223>  Synthetic peptide

<400>  14

Gln Leu Val Gly Thr Asp Tyr Tyr Gly Thr Asp Tyr
1               5                   10

<210>  15
<211>  5
<212>  PRT
<213>  Artificial

<220>
<223>  Synthetic peptide

<400>  15

Ser Phe Ala Met Ser
1               5

<210>  16
<211>  17
<212>  PRT
<213>  Artificial

<220>
<223>  Synthetic peptide

<400>  16

Glu Ile Ser Arg Gly Gly Gly Tyr Ala Phe Tyr Ser Asp Thr Val Thr
1               5                   10                  15

Gly

<210> 17
<211> 12
<212> PRT
<213> Artificial

<220>
<223> Synthetic peptide

<400> 17

Gln Leu Thr Gly Thr Asp Tyr Tyr Ala Met Asp Tyr
1               5                   10

<210> 18
<211> 5
<212> PRT
<213> Artificial

<220>
<223> Synthetic peptide

<400> 18

Arg Tyr Ala Met Ser
1               5

<210> 19
<211> 17
<212> PRT
<213> Artificial

<220>
<223> Synthetic peptide

<400> 19

Glu Ile Asn Ser Gly Gly Ser Tyr Ser Phe Tyr Ser Asp Thr Val Thr
1               5                   10                  15

Gly

<210> 20
<211> 12
<212> PRT
<213> Artificial

<220>
<223> Synthetic peptide

<400> 20

Gln Leu Ser Gly Thr Asp Tyr Tyr Gly Thr Asp Tyr
1               5                   10

<210>  21
<211>  5
<212>  PRT
<213>  Artificial

<220>
<223>  Synthetic peptide

<400>  21

Asn Tyr Ala Met His
1               5


<210>  22
<211>  17
<212>  PRT
<213>  Artificial

<220>
<223>  Synthetic peptide

<400>  22

Trp Ile Asn Thr Tyr Thr Gly Glu Pro Thr Phe Ala Asp Asp Phe Lys
1               5                   10                  15


Gly



<210>  23
<211>  12
<212>  PRT
<213>  Artificial

<220>
<223>  Synthetic peptide

<400>  23

Glu Gly Tyr Tyr Asp Arg Ser His Tyr Phe Asp Tyr
1               5                   10


<210>  24
<211>  16
<212>  PRT
<213>  Artificial

<220>
<223>  Synthetic peptide

<400>  24

Arg Ser Ser Gln Ser Ile Val Tyr Ser Asn Gly Asn Thr Tyr Leu Glu
1               5                   10                  15


<210>  25

<211>  7
<212>  PRT
<213>  Artificial

<220>
<223>  Synthetic peptide

<400>  25

Lys Val Ser Asn Arg Phe Ser
1               5


<210>  26
<211>  9
<212>  PRT
<213>  Artificial

<220>
<223>  Synthetic peptide

<400>  26

Phe Gln Gly Ser His Leu Pro Tyr Thr
1               5


<210>  27
<211>  16
<212>  PRT
<213>  Artificial

<220>
<223>  Synthetic peptide

<400>  27

Arg Ser Ser Gln Asn Ile Val Tyr Ser Asn Gly Asn Thr Tyr Leu Glu
1               5                   10                  15


<210>  28
<211>  9
<212>  PRT
<213>  Artificial

<220>
<223>  Synthetic peptide

<400>  28

Phe Gln Gly Ser His Val Pro Tyr Thr
1               5


<210>  29
<211>  16
<212>  PRT
<213>  Artificial

<220>
<223>  Synthetic peptide

<400>  29

Arg Ser Ser Gln Ser Ile Val Tyr Thr Asn Gly Asn Thr Tyr Leu Glu
1               5                   10                  15


<210>  30
<211>  9
<212>  PRT
<213>  Artificial

<220>
<223>  Synthetic peptide

<400>  30

Phe Gln Gly Ser Gln Val Pro Tyr Thr
1               5


<210>  31
<211>  16
<212>  PRT
<213>  Artificial

<220>
<223>  Synthetic peptide

<400>  31

Arg Ser Ser Gln Ser Leu Leu Tyr Ser Asn Gly Asn Thr Tyr Leu Glu
1               5                   10                  15


<210>  32
<211>  16
<212>  PRT
<213>  Artificial

<220>
<223>  Synthetic peptide

<400>  32

Arg Ser Ser Leu Ser Leu Val His Gly Asp Gly Asn Thr Tyr Leu Glu
1               5                   10                  15


<210>  33
<211>  9
<212>  PRT
<213>  Artificial

<220>
<223>  Synthetic peptide

<400>  33

Leu Gln Gly Ser His Ile Pro Phe Thr
1               5


<210>  34

<211>  121
<212>  PRT
<213>  Artificial

<220>
<223>  Synthetic peptide

<400>  34

Glu Val Gln Leu Val Glu Ser Gly Gly Asp Leu Val Lys Pro Gly Gly
1               5                   10                  15

Ser Leu Lys Leu Ser Cys Ala Ala Ser Gly Phe Thr Phe Ser Ser Tyr
            20                  25                  30

Ala Met Ser Trp Val Arg Gln Ser Pro Glu Lys Arg Leu Glu Trp Val
        35                  40                  45

Ala Glu Ile Ser Gly Ser Gly Ser Arg Asp His Tyr Thr Asp Ser Val
    50                  55                  60

Thr Gly Arg Phe Thr Val Ser Arg Asp Asn Ala Lys Asn Thr Leu Tyr
65                  70                  75                  80

Leu Glu Met Ser Ser Leu Arg Ser Glu Asp Thr Ala Ile Tyr Tyr Cys
                85                  90                  95

Ala Arg Gln Leu Thr Gly Thr Asp Tyr Tyr Gly Thr Asp Tyr Trp Gly
            100                 105                 110

Gln Gly Thr Ser Val Thr Val Ser Ser
        115                 120


<210>  35
<211>  112
<212>  PRT
<213>  Artificial

<220>
<223>  Synthetic peptide

<400>  35

Asp Val Leu Met Thr Gln Ile Pro Leu Ser Leu Pro Val Ser Leu Gly
1               5                   10                  15

Asp Gln Ala Ser Ile Ser Cys Arg Ser Ser Gln Ser Ile Val Tyr Ser
            20                  25                  30

Asn Gly Asn Thr Tyr Leu Glu Trp Tyr Leu Gln Arg Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser His Leu Pro Tyr Thr Phe Gly Gly Gly Thr Lys Leu Glu Ile Lys
            100                 105                 110

<210>  36
<211>  121
<212>  PRT
<213>  Artificial

<220>
<223>  Synthetic peptide

<400>  36

Ala Val Gln Leu Val Glu Ser Gly Gly Gly Leu Val Lys Pro Gly Arg
1               5                   10                  15

Ser Leu Lys Leu Ser Cys Ala Ala Ser Gly Phe Thr Phe Ser Ser Tyr
            20                  25                  30

Ala Met Ser Trp Val Arg Gln Phe Pro Asp Lys Arg Leu Asp Trp Val
        35                  40                  45

Ala Glu Ile Ser Ser Gly Gly Gly Ser Thr Asn Tyr Leu Asp Thr Val
    50                  55                  60

Thr Gly Arg Phe Thr Ile Ser Arg Asp Asn Ala Lys Asn Thr Leu Tyr
65                  70                  75                  80

Leu Glu Met Asn Ser Leu Arg Ser Glu Asp Thr Ala Met Tyr Tyr Cys
                85                  90                  95

Ala Arg Gln Leu Val Gly Thr Asp Tyr Tyr Gly Thr Asp Tyr Trp Gly
            100                 105                 110

Gln Gly Thr Ser Val Thr Val Ser Ser
        115                 120

<210>  37
<211>  112
<212>  PRT
<213>  Artificial

<220>
<223>  Synthetic peptide

<400>  37

Asp Val Leu Met Thr Gln Thr Pro Leu Ser Leu Pro Val Ser Leu Gly
1               5                   10                  15

Asp Gln Ala Ser Ile Ser Cys Arg Ser Ser Gln Asn Ile Val Tyr Ser
            20                  25                  30

Asn Gly Asn Thr Tyr Leu Glu Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser His Val Pro Tyr Thr Phe Gly Gly Gly Thr Lys Leu Glu Ile Lys
            100                 105                 110


<210>  38
<211>  121
<212>  PRT
<213>  Artificial

<220>
<223>  Synthetic peptide

<400>  38

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Phe
            20                  25                  30

Ala Met Ser Trp Val Arg Gln Ser Pro Glu Arg Arg Leu Glu Trp Val
        35                  40                  45

Ala Glu Ile Ser Arg Gly Gly Gly Tyr Ala Phe Tyr Ser Asp Thr Val
    50                  55                  60

Thr Gly Arg Phe Thr Ile Ser Arg Asp Asn Ala Arg Asn Thr Leu Phe
65                  70                  75                  80

Leu Glu Met Gly Ser Leu Arg Ser Glu Asp Thr Ala Met Tyr Tyr Cys
                85                  90                  95

Ser Arg Gln Leu Thr Gly Thr Asp Tyr Tyr Ala Met Asp Tyr Trp Gly
            100                 105                 110

Gln Gly Thr Ser Val Thr Val Ser Ser
        115                 120

<210>  39
<211>  112
<212>  PRT
<213>  Artificial

<220>
<223>  Synthetic peptide

<400>  39

Asp Val Leu Leu Thr Gln Thr Pro Leu Ser Leu Phe Val Ser Leu Gly
1               5                   10                  15

Asp Gln Ala Ser Ile Ser Cys Arg Ser Ser Gln Ser Ile Val Tyr Thr
            20                  25                  30

Asn Gly Asn Thr Tyr Leu Glu Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser Gln Val Pro Tyr Thr Phe Gly Gly Gly Thr Lys Leu Glu Leu Lys
            100                 105                 110

<210>  40
<211>  121
<212>  PRT
<213>  Artificial

<220>
<223>  Synthetic peptide

<400>  40

Glu Val Gln Leu Val Glu Ser Gly Gly Gly Leu Val Lys Pro Gly Gly
1               5                   10                  15

Ser Leu Lys Leu Ser Cys Ser Ala Ser Gly Phe Thr Phe Ser Arg Tyr
            20                  25                  30

Ala Met Ser Trp Val Arg Gln Ser Pro Glu Lys Arg Leu Glu Trp Val
        35                  40                  45

Ala Glu Ile Asn Ser Gly Gly Ser Tyr Ser Phe Tyr Ser Asp Thr Val
    50                  55                  60

Thr Gly Arg Phe Thr Ile Ser Arg Asp Asn Ala Lys Asn Thr Leu Phe
65                  70                  75                  80

Leu Glu Met Ser Ser Leu Arg Ser Glu Asp Thr Ala Ile Tyr Tyr Cys
                85                  90                  95

Ala Arg Gln Leu Thr Gly Thr Asp Tyr Tyr Gly Thr Asp Tyr Trp Gly
            100                 105                 110

Gln Gly Thr Ser Val Thr Val Ser Ser
        115                 120

<210>  41
<211>  112
<212>  PRT
<213>  Artificial

<220>
<223>  Synthetic peptide

<400>  41

Asp Val Leu Met Thr Gln Thr Pro Leu Ser Leu Pro Val Ser His Gly
1               5                   10                  15

Asp Gln Ala Ser Ile Ser Cys Arg Ser Ser Gln Ser Leu Leu Tyr Ser
            20                  25                  30

Asn Gly Asn Thr Tyr Leu Glu Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser His Val Pro Tyr Thr Phe Gly Gly Gly Thr Lys Leu Glu Ile Lys
            100                 105                 110

<210>  42
<211>  121
<212>  PRT
<213>  Artificial

<220>
<223>  Synthetic peptide

<400>  42

Glu Val Gln Leu Val Glu Ser Gly Gly Gly Leu Val Lys Pro Gly Gly
1               5                   10                  15

Ser Leu Lys Leu Ser Cys Ser Ala Ser Gly Phe Thr Phe Ser Arg Tyr
            20                  25                  30

Ala Met Ser Trp Val Arg Gln Ser Pro Glu Lys Arg Leu Glu Trp Val
        35                  40                  45

Ala Glu Ile Asn Ser Gly Gly Ser Tyr Ser Phe Tyr Ser Asp Thr Val
    50                  55                  60

Thr Gly Arg Phe Thr Ile Ser Arg Asp Asn Ala Lys Asn Thr Leu Phe
65                  70                  75                  80

Leu Glu Met Ser Ser Leu Arg Ser Glu Asp Thr Ala Ile Tyr Tyr Cys
                85                  90                  95

Ala Arg Gln Leu Ser Gly Thr Asp Tyr Tyr Gly Thr Asp Tyr Trp Gly
            100                 105                 110

Gln Gly Thr Ser Val Thr Val Ser Ser
        115                 120

<210>  43
<211>  121
<212>  PRT
<213>  Artificial

<220>
<223>  Synthetic peptide

<400>  43

Gln Ile Gln Leu Val Gln Ser Gly Pro Asp Leu Lys Lys Pro Gly Glu
1               5                   10                  15

Thr Val Lys Ile Ser Cys Lys Ala Ser Gly Tyr Thr Phe Thr Asn Tyr
            20                  25                  30

Ala Met His Trp Met Lys Gln Ala Pro Gly Lys Ala Leu Lys Trp Met
        35                  40                  45

Gly Trp Ile Asn Thr Tyr Thr Gly Glu Pro Thr Phe Ala Asp Asp Phe
    50                  55                  60

Lys Gly Arg Phe Ala Phe Ser Leu Glu Thr Ser Ala Ser Thr Ala Tyr
65                  70                  75                  80

Leu Gln Ile Asn Ser Leu Lys Asn Glu Asp Met Ala Thr Tyr Phe Cys
                85                  90                  95

Ala Arg Glu Gly Tyr Tyr Asp Arg Ser His Tyr Phe Asp Tyr Trp Gly
            100                 105                 110

Gln Gly Thr Thr Leu Thr Val Ser Ser
        115                 120

<210>  44
<211>  112
<212>  PRT
<213>  Artificial

<220>
<223>  Synthetic peptide

<400>  44

Asp Val Leu Met Thr Gln Thr Pro Leu Ser Leu Pro Val Ser Leu Gly
1               5                   10                  15

Asp Gln Ala Ser Ile Ser Cys Arg Ser Ser Leu Ser Leu Val His Gly
            20                  25                  30

Asp Gly Asn Thr Tyr Leu Glu Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Leu Gln Gly
                85                  90                  95

Ser His Ile Pro Phe Thr Phe Gly Ser Gly Thr Lys Leu Glu Ile Lys
            100                 105                 110

<210>  45
<211>  299
<212>  PRT
<213>  Homo sapiens

<400>  45

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu Asp Glu Val Lys Glu Gln Val Ala Glu Val Arg
225                 230                 235                 240

Ala Lys Leu Glu Glu Gln Ala Gln Gln Ile Arg Leu Gln Ala Glu Ala
                245                 250                 255

Phe Gln Ala Arg Leu Lys Ser Trp Phe Glu Pro Leu Val Glu Asp Met
            260                 265                 270

Gln Arg Gln Trp Ala Gly Leu Val Glu Lys Val Gln Ala Ala Val Gly
        275                 280                 285

Thr Ser Ala Ala Pro Val Pro Ser Asp Asn His
    290                 295

<210>  46
<211>  76
<212>  PRT
<213>  Artificial

<220>
<223>  Synthetic peptide

<400>  46

Arg Leu Ala Val Tyr Gln Ala Gly Ala Arg Glu Gly Ala Glu Arg Gly
1               5                   10                  15

Leu Ser Ala Ile Arg Glu Arg Leu Gly Pro Leu Val Glu Gln Gly Arg
            20                  25                  30

Val Arg Ala Ala Thr Val Gly Ser Leu Ala Gly Gln Pro Leu Gln Glu
        35                  40                  45

Arg Ala Gln Ala Trp Gly Glu Arg Leu Arg Ala Arg Met Glu Glu Met
    50                  55                  60

Gly Ser Arg Thr Arg Asp Arg Leu Asp Glu Val Lys
65                  70                  75

<210>  47
<211>  34
<212>  PRT
<213>  Artificial

<220>
<223>  Synthetic peptide

<400>  47

Gly Gln Pro Leu Gln Glu Arg Ala Gln Ala Trp Gly Glu Arg Leu Arg
1               5                   10                  15

Ala Arg Met Glu Glu Met Gly Ser Arg Thr Arg Asp Arg Leu Asp Glu
            20                  25                  30

Val Lys

<210>  48
<211>  110
<212>  PRT
<213>  Artificial

<220>
<223>  Synthetic peptide

<400>  48

Gly Gln Pro Leu Gln Glu Arg Ala Gln Ala Trp Gly Glu Arg Leu Arg
1               5                   10                  15

Ala Arg Met Glu Glu Met Gly Ser Arg Thr Arg Asp Arg Leu Asp Glu
            20                  25                  30

Val Lys Glu Gln Val Ala Glu Val Arg Ala Lys Leu Glu Glu Gln Ala
        35                  40                  45

Gln Gln Ile Arg Leu Gln Ala Glu Ala Phe Gln Ala Arg Leu Lys Ser
    50                  55                  60

Trp Phe Glu Pro Leu Val Glu Asp Met Gln Arg Gln Trp Ala Gly Leu
65                  70                  75                  80

Val Glu Lys Val Gln Ala Ala Val Gly Thr Ser Ala Ala Pro Val Pro
                85                  90                  95

Ser Asp Asn His His His His His His His His His His His
            100                 105                 110

<210>  49
<211>  309
<212>  PRT
<213>  Artificial

<220>
<223>  Synthetic peptide

<400>  49

His His His His His His His His His His Lys Val Glu Gln Ala Val
1               5                   10                  15

Glu Thr Glu Pro Glu Pro Glu Leu Arg Gln Gln Thr Glu Trp Gln Ser
            20                  25                  30

Gly Gln Arg Trp Glu Leu Ala Leu Gly Arg Phe Trp Asp Tyr Leu Arg
        35                  40                  45

Trp Val Gln Thr Leu Ser Glu Gln Val Gln Glu Glu Leu Leu Ser Ser
    50                  55                  60

Gln Val Thr Gln Glu Leu Arg Ala Leu Met Asp Glu Thr Met Lys Glu
65                  70                  75                  80

Leu Lys Ala Tyr Lys Ser Glu Leu Glu Glu Gln Leu Thr Pro Val Ala
                85                  90                  95

Glu Glu Thr Arg Ala Arg Leu Ser Lys Glu Leu Gln Ala Ala Gln Ala
            100                 105                 110

Arg Leu Gly Ala Asp Met Glu Asp Val Arg Gly Arg Leu Val Gln Tyr
        115                 120                 125

Arg Gly Glu Val Gln Ala Met Leu Gly Gln Ser Thr Glu Glu Leu Arg
    130                 135                 140

Val Arg Leu Ala Ser His Leu Arg Lys Leu Arg Lys Arg Leu Leu Arg
145                 150                 155                 160

Asp Ala Asp Asp Leu Gln Lys Arg Leu Ala Val Tyr Gln Ala Gly Ala
                165                 170                 175

Arg Glu Gly Ala Glu Arg Gly Leu Ser Ala Ile Arg Glu Arg Leu Gly
            180                 185                 190

Pro Leu Val Glu Gln Gly Arg Val Arg Ala Ala Thr Val Gly Ser Leu
        195                 200                 205

Ala Gly Gln Pro Leu Gln Glu Arg Ala Gln Ala Trp Gly Glu Arg Leu
    210                 215                 220

Arg Ala Arg Met Glu Glu Met Gly Ser Arg Thr Arg Asp Arg Leu Asp
225                 230                 235                 240

Glu Val Lys Glu Gln Val Ala Glu Val Arg Ala Lys Leu Glu Glu Gln
                245                 250                 255

Ala Gln Gln Ile Arg Leu Gln Ala Glu Ala Phe Gln Ala Arg Leu Lys
            260                 265                 270

Ser Trp Phe Glu Pro Leu Val Glu Asp Met Gln Arg Gln Trp Ala Gly

275                 280                 285

Leu Val Glu Lys Val Gln Ala Ala Val Gly Thr Ser Ala Ala Pro Val
    290                 295                 300

Pro Ser Asp Asn His
305

<210>  50
<211>  8
<212>  PRT
<213>  Artificial

<220>
<223>  Synthetic peptide

<220>
<221>  MISC_FEATURE
<222>  (7)..(7)
<223>  Acp

<400>  50

Gly Gln Pro Leu Gln Glu Xaa Cys
1               5

<210>  51
<211>  8
<212>  PRT
<213>  Artificial

<220>
<223>  Synthetic peptide

<220>
<221>  MISC_FEATURE
<222>  (7)..(7)
<223>  Acp

<400>  51

Ala Gly Gln Pro Leu Gln Xaa Cys
1               5

<210>  52
<211>  8
<212>  PRT
<213>  Artificial

<220>
<223>  Synthetic peptide

<220>
<221>  MISC_FEATURE
<222>  (7)..(7)

<223>  Acp

<400>  52

Leu Ala Gly Gln Pro Leu Xaa Cys
1               5


<210>  53
<211>  17
<212>  PRT
<213>  Artificial

<220>
<223>  Synthetic peptide


<220>
<221>  MISC_FEATURE
<222>  (16)..(16)
<223>  Acp

<400>  53

Ala Ala Thr Val Gly Ser Leu Ala Gly Gln Pro Leu Gln Glu Arg Xaa
1               5                   10                  15


Cys




<210>  54
<211>  10
<212>  PRT
<213>  Artificial

<220>
<223>  Synthetic peptide


<220>
<221>  MISC_FEATURE
<222>  (9)..(9)
<223>  Acp

<400>  54

Leu Ala Gly Gln Pro Leu Gln Glu Xaa Cys
1               5                   10


<210>  55
<211>  9
<212>  PRT
<213>  Artificial

<220>
<223>  Synthetic peptide


<220>
<221>  MISC_FEATURE

<222>  (8)..(8)
<223>  Acp

<400>  55

Ala Gly Gln Pro Leu Gln Glu Xaa Cys
1               5

## Description

### Field of the invention

The present invention relates to an antibody or antigen binding portion thereof, which binds to a neo-epitope of a C-terminal fragment of apolipoprotein E, to methods of producing such an antibody or antigen binding portion thereof, and to therapeutic and diagnostic uses thereof.

### Background

Alzheimer's disease (AD) is a progressive neurodegenerative dementia disorder, which exists in a more common late-onset form and an early-onset familial form. AD is characterized by progressive loss of memory and cognitive function. At present, AD treatments are limited to symptomatic management and the prognosis is poor for AD patients. It is estimated that about 18 million people worldwide are presently suffering from AD, and the number of people suffering from AD is expected to increase due to the aging population. The prevalence of AD doubles approximately every 5 years from the age of 60, from 10% of individuals at the age of 65 to 50% of individuals at the age of 85 or more (Solomon, Expert Opin. Investig. Drugs (2007) 16(6): 819-828).

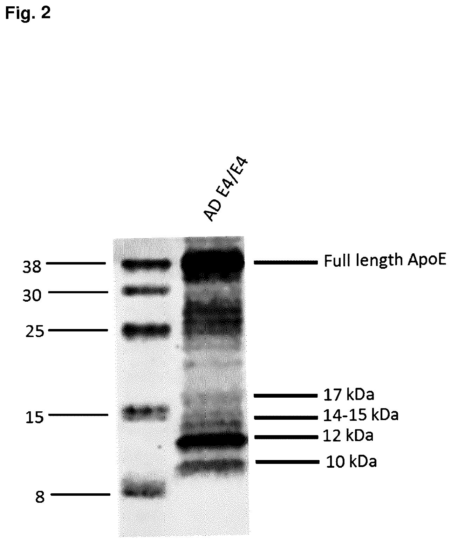

A known genetic risk factor for late-onset AD is the APOE ε4 allele, although its precise role in the disease remains unclear. The APOE gene encodes apolipoprotein E (ApoE), which is a glycoprotein of 35 kDa expressed at high levels in the brain. ApoE exists in three different isoforms, ApoE2, ApoE3 and ApoE4, of which ApoE3 is the most common, ApoE2 has been shown to decrease the risk of AD, and ApoE4 increases it. Although it is not clear how ApoE contributes to the onset and progression of AD, several studies have shown that the protein is subject to fragmentation in the human brain. The specific functions of ApoE fragments and their possible links with AD are reviewed by Muñoz et al in Neurochem Res (2019) 44(6): 1297-1305. With regards to the neurotoxicity of ApoE fragments, Muñoz *et al* conclude that the existing research shows that both the N-terminal LDL-binding domain and the C-terminal lipid-binding domain of ApoE are necessary for a neurotoxic effect (see e.g. page 1300 lines 4-8). With regards to ApoE fragments from the C-terminal, lipid-binding domain, Table 2 and Figure 2 of Muñoz *et al* indicate that one such fragment has been studied previously and shown to have a stabilizing effect on hexamers of amyloid beta (Aβ) peptide. The study in question was reported by Wellnitz et al in J Neurochem (2005) 94: 1351-1360, and discloses a 13 kDa fragment of ApoE with an N-terminal start at amino acid position 187 of ApoE. No antibodies specific for this fragment are disclosed, but it is studied using an antibody designated 3H1. The antibody 3H1 is disclosed as binding to an epitope in the C-terminal part of ApoE formed by amino acid residues 243-273.

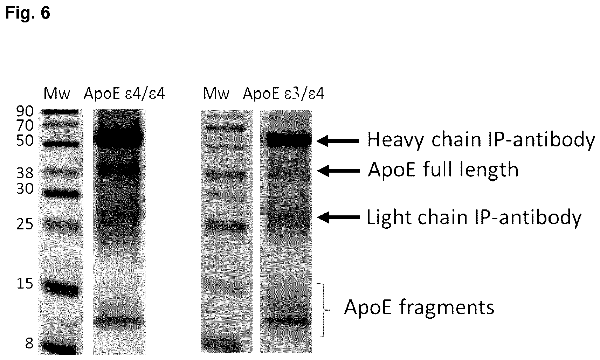

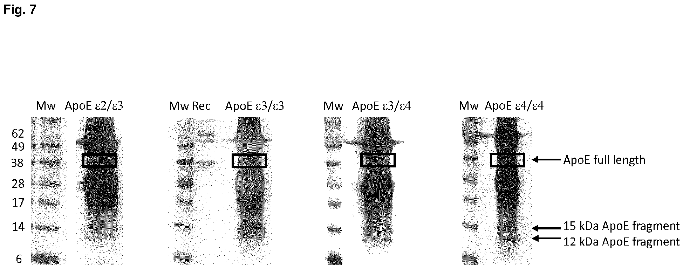

A similar conclusion regarding the neurotoxicity of ApoE fragments was reached in an earlier review by Mahley and Huang in Neuron (2012) 76: 871-885. This article focuses primarily on the potential neurotoxicity of fragments of the specific isoform ApoE4. Whereas it does show that an ApoE4 fragment of 12 kDa is generated (see e.g. Figure 6), the conclusion is drawn that neurotoxic ApoE fragments comprise both the LDL receptor binding region (aa 136-150) and the lipid binding region (aa 240-270) (see e.g. Figure 7 and page 876, right col, lines 13-21). Mahley and Huang do not disclose any antibodies specific for the reviewed ApoE4 fragments.

Finally, a study by Mouchard et al in Sci Rep (2019) 9(1): 3989 investigated the association between fragments of ApoE and amyloid beta (Aβ) peptides of different sizes. An ApoE fragment of 12 kDa was identified, but was found to not interact with Aβ. The antibodies used in the Mouchard *et al* study are listed in Supplementary Table 2, and do not include any antibody specific for the 12 kDa ApoE fragment.

There remains a need in the art for novel therapeutic and/or diagnostic tools for detecting and treating Alzheimer's disease. There is also a need to further elucidate and characterize the role of ApoE fragments in the onset and progression of neurodegenerative disease.

### Summary of the invention

The present invention relates to antibodies and antigen binding portions thereof that bind to apolipoprotein E (ApoE). As reported herein, the antibodies and antigen binding portions of the invention bind to a neo-epitope present within C-terminal fragments of ApoE.

Thus, in a first aspect, the present invention provides an antibody or antigen binding portion thereof that binds to a fragment of apolipoprotein E (ApoE), wherein the fragment has
- an apparent molecular weight of 12 kDa as measured by SDS-PAGE, and
- an N-terminus corresponding to an amino acid in full-length apolipoprotein E which is selected from the group consisting of amino acids L198, A199 and G200; and wherein the antibody or antigen binding portion thereof binds to an epitope comprising the N-terminus of the fragment. In certain embodiments, the ApoE fragment consists of the amino acid sequence of any one of SEQ ID NOs: 1, 2 or 3.

In a further aspect, the present invention provides methods of producing antibodies or antigen binding portions thereof, the methods comprising a step of immunizing a host mammal with a peptide immunogen comprising an N-terminal amino acid sequence selected from the group consisting of LAGQPL (SED ID NO:4), AGQPLQ (SEQ ID NO:5), GQPLQE (SEQ ID NO:6), LAGQPLQ (SEQ ID NO:7), AGQPLQE (SEQ ID NO:8) and LAGQPLQE (SEQ ID NO:9). Antibodies and antigen binding portions thereof obtainable by said methods are encompassed by a third aspect of the invention.

In a still further aspect, the present invention provides a pharmaceutical composition comprising an antibody or antigen binding portion thereof in accordance with the first or third aspect of the invention and a pharmaceutically acceptable excipient or carrier.

In further aspects, the present invention provides antibodies, antigen binding portions thereof and/or pharmaceutical compositions comprising the same for use in methods of treatment or for use in methods of detection or diagnosis as described herein.

The invention can be further understood with reference to the following illustrative embodiments.
1. An antibody or antigen binding portion thereof that binds to a fragment of apolipoprotein E (ApoE), wherein the fragment has
   - an apparent molecular weight of 12 kDa as measured by SDS-PAGE, and
   - an N-terminus corresponding to an amino acid in full-length apolipoprotein E which is selected from the group consisting of amino acids L198, A199 and G200; and wherein the antibody or antigen binding portion thereof binds to an epitope comprising the N-terminus of the fragment.
2. The antibody or antigen binding portion thereof according to (1), wherein the antibody or antigen binding portion thereof binds selectively to the ApoE fragment.
3. The antibody or antigen binding portion thereof according to (1) or (2), wherein the antibody or antigen binding portion thereof does not bind to full-length apolipoprotein E.
4. The antibody or antigen binding portion thereof according to any one of (1)-(3), wherein the fragment of apolipoprotein E has an N-terminus corresponding to amino acid G200 in full-length apolipoprotein E.
5. The antibody or antigen binding portion thereof according to any one of (1)-(4), wherein the antibody or antigen binding portion thereof binds to an epitope comprising amino acid residues 200-205 in full-length apolipoprotein E (GQPLQE).
6. The antibody or antigen binding portion thereof according to any one of (1)-(5), wherein the fragment of apolipoprotein E is selected from:
   i) a fragment consisting of the amino acid sequence of any one of SEQ ID NOs:1-3; and
   ii) a fragment having at least 80% identity to any one of SEQ ID NOs: 1-3.
7. The antibody or antigen binding portion thereof according to (6), wherein the fragment of apolipoprotein E is selected from a fragment consisting of the amino acid sequence of any one of SEQ ID NOs:1, 2 and 3.
8. The antibody or antigen binding portion thereof according to (6), wherein the fragment of apolipoprotein E consists of the amino acid sequence of SEQ ID NO: 1.
9. The antibody or antigen binding portion thereof according to (6), wherein the fragment of apolipoprotein E consists of the amino acid sequence of SEQ ID NO: 2.
10. The antibody or antigen binding portion thereof according to (9), wherein the antibody or antigen binding portion thereof binds to an epitope comprising amino acid residues 199-204 in full-length apolipoprotein E (AGQPLQ) or amino acid residues 199-205 in full-length apolipoprotein E (AGQPLQE).
11. The antibody or antigen binding portion thereof according to (6), wherein the fragment of apolipoprotein E consists of the amino acid sequence of SEQ ID NO: 3.
12. The antibody or antigen binding portion thereof according to (11), wherein the antibody or antigen binding portion thereof binds to an epitope comprising:
   - amino acid residues 198-203 in full-length apolipoprotein E (LAGQPL);
   - amino acid residues 198-204 in full-length apolipoprotein E (LAGQPLQ); or
   - amino acid residues 198-205 in full-length apolipoprotein E (LAGQPLQE).
13. A method of producing an antibody or an antigen binding portion thereof, comprising a step of immunizing a suitable host mammal with a peptide immunogen comprising an N-terminal amino acid sequence selected from the group consisting of LAGQPL (SED ID NO:4), AGQPLQ (SEQ ID NO:5), GQPLQE (SEQ ID NO:6), LAGQPLQ (SEQ ID NO:7), AGQPLQE (SEQ ID NO:8) and LAGQPLQE (SEQ ID NO:9).
14. The method according to (13), wherein said N-terminal amino acid sequence is GQPLQE (SEQ ID NO:6).
15. The method according to (13), wherein said N-terminal amino acid sequence is selected from LAGQPL (SED ID NO:4), LAGQPLQ (SEQ ID NO:7) and LAGQPLQE (SEQ ID NO:9).
16. The method according to (13), wherein said N-terminal amino acid sequence is selected from AGQPLQ (SEQ ID NO:5) and AGQPLQE (SEQ ID NO:8).
17. An antibody or antigen binding portion thereof, obtainable by a method according to any one of (13)-(16).
18. An antibody or antigen binding portion thereof according to any one of (1)-(12) or (17), wherein the antibody or antigen binding portion thereof comprises a variable heavy chain domain (VH) comprising three CDR sequences (CDR-H1, CDR-H2 and CDR-H3), wherein the three VH CDR sequences are independently selected from:
   - CDR-H1 selected from the group consisting of SEQ ID NO: 10, 15, 18 and 21;
   - CDR-H2 selected from the group consisting of SEQ ID NO: 11, 13, 16, 19 and 22; and
   - CDR-H3 selected from the group consisting of SEQ ID NO: 12, 14, 17, 20 and 23.
19. An antibody or antigen binding portion thereof according to any one of (1)-(12), (17) or (18), wherein the antibody or antigen binding portion thereof comprises a variable light chain domain (VL) comprising three CDR sequences (CDR-L1, CDR-L2 and CDR-L3), wherein the three VL CDR sequences are independently selected from:
   - CDR-L1 selected from the group consisting of SEQ ID NO: 24, 27, 29, 31 and 32;
   - CDR-L2 being SEQ ID NO: 25; and
   - CDR-L3 selected from the group consisting of SEQ ID NO: 26, 28, 30 and 33.
20. An antibody or antigen binding portion thereof that binds to apolipoprotein E (ApoE), wherein the antibody or antigen binding portion thereof comprises a variable heavy chain domain (VH) comprising three CDR sequences (CDR-H1, CDR-H2 and CDR-H3), wherein the three VH CDR sequences are independently selected from:
   - CDR-H1 selected from the group consisting of SEQ ID NO: 10, 15, 18 and 21;
   - CDR-H2 selected from the group consisting of SEQ ID NO: 11, 13, 16, 19 and 22; and
   - CDR-H3 selected from the group consisting of SEQ ID NO: 12, 14, 17, 20 and 23.
21. The antibody or antigen binding portion thereof according to (20), wherein the antibody or antigen binding portion thereof additionally comprises a variable light chain domain (VL) comprising three CDR sequences (CDR-L1, CDR-L2 and CDR-L3), wherein the three VL CDR sequences are independently selected from:
   - CDR-L1 selected from the group consisting of SEQ ID NO: 24, 27, 29, 31 and 32;
   - CDR-L2 being SEQ ID NO: 25; and
   - CDR-L3 selected from the group consisting of SEQ ID NO: 26, 28, 30 and 33.
22. The antibody or antigen binding portion thereof according to any one of (18)-(21), wherein the antibody or antigen binding portion thereof comprises the CDR sequences:
   CDR-H1 comprising or consisting of SEQ ID NO: 10 (SYAMS);
   CDR-H2 comprising or consisting of SEQ ID NO: 11 (EISGSGSRDHYTDSVTG) ;
   CDR-H3 comprising or consisting of SEQ ID NO: 12 (QLTGTDYYGTDY);
   CDR-L1 comprising or consisting of SEQ ID NO: 24 (RSSQSIVYSNGNTYLE);
   CDR-L2 comprising or consisting of SEQ ID NO: 25 (KVSNRFS); and
   CDR-L3 comprising or consisting of SEQ ID NO: 26 (FQGSHLPYT).
23. The antibody or antigen binding portion thereof according to any one of (18)-(21), wherein the antibody or antigen binding portion thereof comprises the CDR sequences:
   CDR-H1 comprising or consisting of SEQ ID NO: 10 (SYAMS);
   CDR-H2 comprising or consisting of SEQ ID NO: 13 (EISSGGGSTNYLDTVTG) ;
   CDR-H3 comprising or consisting of SEQ ID NO: 14 (QLVGTDYYGTDY);
   CDR-L1 comprising or consisting of SEQ ID NO: 27 (RSSQNIVYSNGNTYLE) ;
   CDR-L2 comprising or consisting of SEQ ID NO: 25 (KVSNRFS); and
   CDR-L3 comprising or consisting of SEQ ID NO: 28 (FQGSHVPYT).
24. The antibody or antigen binding portion thereof according to any one of (18)-(21), wherein the antibody or antigen binding portion thereof comprises the CDR sequences:
   CDR-H1 comprising or consisting of SEQ ID NO: 15 (SFAMS);
   CDR-H2 comprising or consisting of SEQ ID NO: 16 (EISRGGGYAFYSDTVTG);
   CDR-H3 comprising or consisting of SEQ ID NO: 17 (QLTGTDYYAMDY);
   CDR-L1 comprising or consisting of SEQ ID NO: 29 (RSSQSIVYTNGNTYLE);
   CDR-L2 comprising or consisting of SEQ ID NO: 25 (KVSNRFS); and
   CDR-L3 comprising or consisting of SEQ ID NO: 30 (FQGSQVPYT).
25. The antibody or antigen binding portion thereof according to any one of (18)-(21), wherein the antibody or antigen binding portion thereof comprises the CDR sequences:
   CDR-H1 comprising or consisting of SEQ ID NO: 18 (RYAMS);
   CDR-H2 comprising or consisting of SEQ ID NO: 19 (EINSGGSYSFYSDTVTG) ;
   CDR-H3 comprising or consisting of SEQ ID NO: 12 (QLTGTDYYGTDY);
   CDR-L1 comprising or consisting of SEQ ID NO: 31 (RSSQSLLYSNGNTYLE);
   CDR-L2 comprising or consisting of SEQ ID NO: 25 (KVSNRFS); and
   CDR-L3 comprising or consisting of SEQ ID NO: 28 (FQGSHVPYT).
26. The antibody or antigen binding portion thereof according to any one of (18)-(21), wherein the antibody or antigen binding portion thereof comprises the CDR sequences:
   CDR-H1 comprising or consisting of SEQ ID NO: 18 (RYAMS);
   CDR-H2 comprising or consisting of SEQ ID NO: 19 (EINSGGSYSFYSDTVTG) ;
   CDR-H3 comprising or consisting of SEQ ID NO: 20 (QLSGTDYYGTDY) ;
   CDR-L1 comprising or consisting of SEQ ID NO: 31 (RSSQSLLYSNGNTYLE);
   CDR-L2 comprising or consisting of SEQ ID NO: 25 (KVSNRFS); and
   CDR-L3 comprising or consisting of SEQ ID NO: 28 (FQGSHVPYT).
27. The antibody or antigen binding portion thereof according to any one of (18)-(21), wherein the antibody or antigen binding portion thereof comprises the CDR sequences:
   CDR-H1 comprising or consisting of SEQ ID NO: 21 (NYAMH);
   CDR-H2 comprising or consisting of SEQ ID NO: 22 (WINTYTGEPTFADDFKG) ;
   CDR-H3 comprising or consisting of SEQ ID NO: 23 (EGYYDRSHYFDY) ;
   CDR-L1 comprising or consisting of SEQ ID NO: 32 (RSSLSLVHGDGNTYLE);
   CDR-L2 comprising or consisting of SEQ ID NO: 25 (KVSNRFS); and
   CDR-L3 comprising or consisting of SEQ ID NO: 33 (LQGSHIPFT).
28. An antibody or antigen binding portion thereof according to any one of (1)-(12) and (17)-(21), wherein the antibody or antigen binding portion thereof comprises a heavy chain variable domain (VH) comprising or consisting of an amino acid sequence selected from:
   i) the group consisting of SEQ ID NOs: 34, 36, 38, 40, 42 and 43; and
   ii) a sequence having at least 70%, at least 80%, at least 90%, or at least 95% identity to any one of SEQ ID NOs: 34, 36, 38, 40, 42 and 43.
29. An antibody or antigen binding portion thereof according to any one of (1)-(12), (17)-(21) or (28), wherein the antibody or antigen binding portion thereof comprises a light chain variable domain (VL) comprising or consisting of an amino acid sequence selected from:
   i) the group consisting of SEQ ID NOs: 35, 37, 39, 41 and 44; and
   ii) a sequence having at least 70%, at least 80%, at least 90%, or at least 95% identity to any one of SEQ ID NO:35, 37, 39, 41 and 44.
30. The antibody or antigen binding portion thereof according to (28) or (29), comprising a variable heavy chain domain (VH) and a variable light chain domain (VL) selected from the group consisting of:
   (i) a VH domain comprising or consisting of the amino acid sequence of SEQ ID NO: 34 or an amino acid sequence having at least 80%, 90%, 95%, 98%, or 99% identity thereto, and a VL domain comprising or consisting of the amino acid sequence of SEQ ID NO: 35 or an amino acid sequence having at least 80%, 90%, 95%, 98%, or 99% identity thereto;
   (ii) a VH domain comprising or consisting of the amino acid sequence of SEQ ID NO: 36 or an amino acid sequence having at least 80%, 90%, 95%, 98%, or 99% identity thereto, and a VL domain comprising or consisting of the amino acid sequence of SEQ ID NO: 37 or an amino acid sequence having at least 80%, 90%, 95%, 98%, or 99% identity thereto;
   (iii) a VH domain comprising or consisting of the amino acid sequence of SEQ ID NO: 38 or an amino acid sequence having at least 80%, 90%, 95%, 98%, or 99% identity thereto, and a VL domain comprising or consisting of the amino acid sequence of SEQ ID NO: 39 or an amino acid sequence having at least 80%, 90%, 95%, 98%, or 99% identity thereto;
   (iv) a VH domain comprising or consisting of the amino acid sequence of SEQ ID NO: 40 or an amino acid sequence having at least 80%, 90%, 95%, 98%, or 99% identity thereto, and a VL domain comprising or consisting of the amino acid sequence of SEQ ID NO: 41 or an amino acid sequence having at least 80%, 90%, 95%, 98%, or 99% identity thereto;
   (v) a VH domain comprising or consisting of the amino acid sequence of SEQ ID NO: 42 or an amino acid sequence having at least 80%, 90%, 95%, 98%, or 99% identity thereto, and a VL domain comprising or consisting of the amino acid sequence of SEQ ID NO: 41 or an amino acid sequence having at least 80%, 90%, 95%, 98%, or 99% identity thereto; and
   (vi) a VH domain comprising or consisting of the amino acid sequence of SEQ ID NO: 43 or an amino acid sequence having at least 80%, 90%, 95%, 98%, or 99% identity thereto, and a VL domain comprising or consisting of the amino acid sequence of SEQ ID NO: 44 or an amino acid sequence having at least 80%, 90%, 95%, 98%, or 99% identity thereto.
31. The antibody or antigen binding portion thereof according to (30), comprising a variable heavy chain domain (VH) and a variable light chain domain (VL) selected from the group consisting of:
   (i) a VH domain comprising or consisting of the amino acid sequence of SEQ ID NO: 34 and a VL domain comprising or consisting of the amino acid sequence of SEQ ID NO: 35;
   (ii) a VH domain comprising or consisting of the amino acid sequence of SEQ ID NO: 36 and a VL domain comprising or consisting of the amino acid sequence of SEQ ID NO: 37;
   (iii) a VH domain comprising or consisting of the amino acid sequence of SEQ ID NO: 38 and a VL domain comprising or consisting of the amino acid sequence of SEQ ID NO: 39;
   (iv) a VH domain comprising or consisting of the amino acid sequence of SEQ ID NO: 40, and a VL domain comprising or consisting of the amino acid sequence of SEQ ID NO: 41;
   (v) a VH domain comprising or consisting of the amino acid sequence of SEQ ID NO: 42, and a VL domain comprising or consisting of the amino acid sequence of SEQ ID NO: 41; and
   (vi) a VH domain comprising or consisting of the amino acid sequence of SEQ ID NO: 43, and a VL domain comprising or consisting of the amino acid sequence of SEQ ID NO: 44.
32. A pharmaceutical composition, comprising an antibody or antigen binding portion thereof according to any one of (1)-(12) or (17)-(31) and at least one pharmaceutically acceptable excipient or carrier.
33. An antibody or antigen binding portion thereof according to any one of (1)-(12) or (17)-(31), or pharmaceutical composition according to (32), for use as a therapeutic, prognostic or diagnostic agent.
34. An antibody or antigen binding portion thereof according to any one of (1)-(12) or (17)-(31), or pharmaceutical composition according to (32), for use as a therapeutic agent.
35. A method of preventing or treating a neurological disorder or a disorder characterized by a loss of cognitive memory capacity in a subject in need thereof, wherein the method comprises administering to the subject an antibody or antigen binding portion thereof according to any one of (1)-(12) or (17)-(31), or pharmaceutical composition according to (32).
36. The method of (35), wherein the disorder is selected from Alzheimer's disease (AD), mild cognitive impairment (MCI), dementia with Lewy body, Down's syndrome, hereditary cerebral hemorrhage with amyloidosis (Dutch type), cerebral amyloid angiopathy, Parkinson's disease, and cataract due to amyloid beta deposition.
37. The method of (36), wherein the disorder is Alzheimer's disease.
38. An antibody or antigen binding portion thereof according to any one of (1)-(12) or (17)-(31), or pharmaceutical composition according to (32) for use in the prevention or treatment of a neurological disorder or a disorder characterized by a loss of cognitive memory capacity.
39. The antibody, antigen binding portion thereof or pharmaceutical composition for use according to (38), wherein the disorder is selected from Alzheimer's disease (AD), mild cognitive impairment (MCI), dementia with Lewy body, Down's syndrome, hereditary cerebral hemorrhage with amyloidosis (Dutch type), cerebral amyloid angiopathy, Parkinson's disease, and cataract due to amyloid beta deposition.
40. The antibody, antigen binding portion thereof or pharmaceutical composition for use according to (39), wherein the disorder is Alzheimer's disease.
41. An antibody or antigen binding portion thereof according to any one of (1)-(12) or (17)-(31), or pharmaceutical composition according to (32), for use as a diagnostic agent.
42. A method of detecting or diagnosing a neurological disorder or a disorder characterized by a loss of cognitive memory capacity in a subject, the method comprising contacting a sample obtained from the subject with an antibody or antigen binding portion thereof according to any one of (1)-(12) or (17)-(31).
43. The method of (42), wherein the disorder is selected from Alzheimer's disease (AD), mild cognitive impairment (MCI), dementia with Lewy body, Down's syndrome, hereditary cerebral hemorrhage with amyloidosis (Dutch type), cerebral amyloid angiopathy, Parkinson's disease, and cataract due to amyloid beta deposition.
44. The method of (43), wherein the disorder is Alzheimer's disease.
45. An antibody or antigen binding portion thereof according to any one of (1)-(12) or (17)-(31), or pharmaceutical composition according to (32) for use in the diagnosis of a neurological disorder or a disorder characterized by a loss of cognitive memory capacity.
46. The antibody, antigen binding portion thereof or pharmaceutical composition for use according to (45), wherein the disorder is selected from Alzheimer's disease (AD), mild cognitive impairment (MCI), dementia with Lewy body, Down's syndrome, hereditary cerebral hemorrhage with amyloidosis (Dutch type), cerebral amyloid angiopathy, Parkinson's disease, and cataract due to amyloid beta deposition.
47. The antibody, antigen binding portion thereof or pharmaceutical composition for use according to (46), wherein the disorder is Alzheimer's disease.

### Brief description of the figures

Figure 1 shows the results of Western blot analysis of human brain extracts as described in Example 1.
Figure 2 shows the results of Western blot analysis of human brain extract from AD brain of genotype APOE ε4/ε4 at sufficiently high resolution to show individual low molecular weight ApoE fragments as described in Example 1.
Figure 3 is a diagram showing the ratio of 12 kDa ApoE fragment to full-length ApoE in AD (filled circles) and control (open squares), quantified as described in Example 1.
Figure 4 is a diagram showing the ratio of 12 kDa ApoE fragment to full-length ApoE in AD without (-E4; filled circles) or with (+E4; open squares) APOE E4 genotype, quantified as described in Example 1.
Figure 5 is a schematic overview of the workflow for the immunoprecipitation experiments described in Example 2.
Figure 6 shows the result of Western blot analysis of immunoprecipitated samples as described in Example 2.
Figure 7 shows the result of silver staining of immunoprecipitated samples as described in Example 2.
Figure 8 shows the result of LC-MS/MS analysis of tryptic digests of 12 kDa, 15 kDa and rhApoE4 gels as indicated, as described in Example 3.
Figure 9 shows the result of LysC cleavage site analysis of the ApoE sequence as described in Example 4.
Figure 10 shows the result of investigation by extracted-ion chromatograms (XIC) of theoretical ApoE cleavage sites as described in Example 5. Left side: Extracted ion chromatograms at theoretical values of three charge states of one of the possible peptides (200-233) with 5 ppm mass accuracy, with peaks observed at the same retention time for all three. Right side: The mass spectrum from each extracted peak.
Figure 11 shows the result of nanoLC-MS/MS with the shotgun proteomic method for detection of peptides around cleavage sites as described in Example 5. In replicate analyses of samples from the same donor (APOE ε3/ε4, A and B), peptides having an N terminus at L198, A199 or G200 and an intact C terminus of ApoE were detected.
Figure 12 is a diagram showing the MS intensity for peptides having an N terminus at L198, A199 or G200 in samples from APOE ε4/ε4, ε2/ε3 and ε3/ε3 carriers as indicated, as described in Example 6.
Figure 13 shows the mitochondrial damage induced by human ApoE4 and ApoE C-terminal fragments following the experiment described in Example 7, in (A) Neuro2A cells and (B) rat primary hippocampal neurons; as well as (C) protein expression of human ApoE4 or ApoE C-terminal fragments as measured by Western blot analysis.
Figure 14 shows concentration-response curves for hybridoma antibodies generated using the G200 neo-epitope, as described in Example 10.
Figure 15 shows the binding of hybridoma antibodies, generated using the G200 neo-epitope, to a brain extract from an Alzheimer's disease patient as described in Example 10.
Figure 16 shows concentration-response curves for recombinant antibodies against the G200 neo-epitope, as described in Example 12.
Figure 17 shows inhibition-response curves for recombinant antibodies against the G200 neo-epitope, as described in Example 12.
Figure 18 shows a sample of binding interactions for recombinant antibodies against the G200 neo-epitope, characterized via bio-layer interferometry as described in Example 12.

### Detailed Description

One object of the invention is to further elucidate the structure of ApoE fragments in the brains of AD patients.

Another object is to provide novel insights into the function that ApoE fragments have in the genesis of disease.

Another object of the invention is to enable therapeutic intervention through targeting of novel epitopes on such ApoE fragments.

Another object of the invention is to enable the diagnosis of AD and other neurodegenerative disorders via detection of ApoE fragments implicated in disease formation and/or progression.

Another object of the invention is to provide antibodies, or antigen binding portions thereof, having a novel and useful binding specificity.

One or more of these and other objects that are apparent to the skilled person from reading the entire disclosure herein are met by the various aspects disclosed.

### Anti-ApoE antibodies and methods of production

Thus, in a first aspect, the disclosure provides an antibody or antigen binding portion that binds to a fragment of apolipoprotein E (ApoE), wherein the fragment has
- an apparent molecular weight of 12 kDa as measured by SDS-PAGE, and
- an N-terminus corresponding to an amino acid in full-length apolipoprotein E which is selected from the group consisting of amino acids L198, A199 and G200; and wherein the antibody or antigen binding portion thereof binds to an epitope comprising the N-terminus of the fragment. The full-length apolipoprotein E from which the fragment derives is typically human ApoE.

The invention is based on detailed insights into the structure of putatively neurotoxic ApoE fragments that were isolated from brains of Alzheimer's disease patients, as detailed in Examples 1-7 which follow. The identification of the exact sequence of these fragments enables the generation of inventive antibodies, or antigen binding portions thereof, that are specific for the N-terminal neo-epitopes formed upon ApoE fragmentation. Generation and characterization of exemplary such antibodies is detailed in Examples 8-14.

Without wishing to be bound by theory, it is contemplated that such novel antibodies, or antigen binding portions thereof, are useful in the diagnosis, prognosis and/or treatment of neurodegenerative diseases such as Alzheimer's disease, through specific binding to the putatively neurotoxic ApoE fragments.

In certain embodiments, the antibodies and antigen binding portions thereof of the first aspect bind selectively to the ApoE fragments described herein. As used herein, the term "bind selectively" refers to the preferential binding of the antibody or antigen binding portion thereof to the ApoE fragment target. In certain embodiments, the antibodies and antigen binding portions thereof of the first aspect do not bind to full-length apolipoprotein E, particularly full-length human apolipoprotein E.

In one embodiment, the antibody or antigen binding portion thereof of the first aspect is capable of selective binding to an epitope comprising the N-terminus of a fragment of apolipoprotein E, which fragment has an N-terminus corresponding to amino acid G200 in full-length ApoE.

In another embodiment, the antibody or antigen binding portion thereof of the first aspect is capable of selective binding to an epitope comprising the N-terminus of a fragment of apolipoprotein E, which fragment is selected from
i) the group consisting of SEQ ID NO:1-3; and
ii) a sequence having at least 80 % identity to any one of SEQ ID NO:1-3.

In a more specific embodiment, i) in this definition is SEQ ID NO:1. In an alternative embodiment, i) in this definition is SEQ ID NO:2. In another embodiment, i) in this definition is SEQ ID NO:3.

As defined in this group of embodiments, the ApoE fragment with the epitope of interest may have a sequence that has at least 80 % sequence identity to a sequence selected from SEQ ID NO:1-3. In one embodiment, said sequence may have at least 85 %, such as at least 90 %, such as at least 95 %, such as 100 %, identity to a sequence selected from SEQ ID NO:1-3. In one embodiment, such variation in the target sequence, to which the antibody or antigen binding portion thereof binds, is subject to the condition that the fragment retains the first three, such as the first four, such as the first five, such as the first six, amino acids of the sequence selected from SEQ ID NO:1-3, starting from the N-terminal, so as to ensure that the N-terminal neo-epitope of the fragment is present.

In the embodiment wherein ii) has 100 % identity to i), the fragment of ApoE consists of an amino acid sequence selected from the group consisting of SEQ ID NO:1-3. In a more specific embodiment, the fragment consists of SEQ ID NO:1. In an alternative embodiment, the fragment consists of SEQ ID NO:2. In another alternative embodiment, the fragment consists of SEQ ID NO:3.

The antibodies and antigen binding portions of the first aspect bind to neo-epitopes at the N-terminus of the ApoE fragments described herein. In one embodiment, the antibody or antigen binding portion thereof binds to an epitope comprising amino acid residues 200-205 in full-length apolipoprotein E (GQPLQE). In one embodiment, the antibody or antigen binding portion thereof binds to an epitope comprising amino acid residues 199-204 in full-length apolipoprotein E (AGQPLQ). In one embodiment, the antibody or antigen binding portion thereof binds to an epitope comprising amino acid residues 199-205 in full-length apolipoprotein E (AGQPLQE). In one embodiment, the antibody or antigen binding portion thereof binds to an epitope comprising amino acid residues 198-203 in full-length apolipoprotein E (LAGQPL). In one embodiment, the antibody or antigen binding portion thereof binds to an epitope comprising amino acid residues 198-204 in full-length apolipoprotein E (LAGQPLQ). In one embodiment, the antibody or antigen binding portion thereof binds to an epitope comprising amino acid residues 198-205 in full-length apolipoprotein E (LAGQPLQE).

In a second aspect of the disclosure, there is provided a method of production of an antibody or an antigen binding portion thereof, comprising a step of immunizing a suitable host mammal with an immunogen that comprises one of the identified neo-epitopes of the putatively neurotoxic ApoE fragments disclosed herein. As such, this method comprises immunizing the host with a peptide immunogen comprising an N-terminal amino acid sequence selected from the group consisting of LAGQPL (SED ID NO:4), AGQPLQ (SEQ ID NO:5), GQPLQE (SEQ ID NO:6), LAGQPLQ (SEQ ID NO:7), AGQPLQE (SEQ ID NO:8) and LAGQPLQE (SEQ ID NO:9). The host mammal is preferably a non-human mammal

In one embodiment of the second aspect, the peptide immunogen comprises an N-terminal amino acid sequence which is GQPLQE (SEQ ID NO:6). In another embodiment, the N-terminal amino acid sequence of the peptide immunogen is selected from LAGQPL (SED ID NO:4), LAGQPLQ (SEQ ID NO:7) and LAGQPLQE (SEQ ID NO:9). In another embodiment, the N-terminal amino acid sequence is selected from AGQPLQ (SEQ ID NO:5) and AGQPLQE (SEQ ID NO:8).

In addition to the step of immunizing a suitable host, the method of the second aspect of the disclosure may suitably comprise additional steps of a standard nature for the generation of antibodies from the immunized animals, such as plasma screening for reactive antibodies, isolation of spleen cells, generation of hybridomas, and other measures known to the person of skill in the art of antibody generation.

In a third aspect of the disclosure, there is provided an antibody or antigen binding portion thereof, which is obtainable by a method according to the second aspect. Such an antibody or antigen binding portion thereof is likely to exhibit the desired selectivity shown by the antibody, or antigen binding portion thereof, of the first aspect, and to be useful in the same contexts of diagnosis, prognosis and treatment of neurodegenerative disease.

In some embodiments of the antibody or antigen binding portion thereof according to the first and third aspects, the antibody or antigen binding portion thereof is selected from the group consisting of full-length antibodies, Fab fragments, Fab' fragments, F(ab')₂ fragments, Fc fragments, Fv fragments, single chain Fv fragments, (scFv)₂ and domain antibodies. In one embodiment, said at least one antibody or antigen binding portion thereof is selected from full-length antibodies, Fab fragments and scFv fragments. In one particular embodiment, the antibody is a full length antibody. In one embodiment, the antibody or antigen binding portion thereof is selected from the group consisting of monoclonal antibodies, human antibodies, humanized antibodies, and antigen binding portions thereof. In another embodiment, it is a monoclonal antibody or antigen binding portion thereof.

In a more specific such embodiment, the antibody or antigen binding portion thereof comprises at least three complementarity determining regions (CDRs).

As the skilled person will realize, embodiments of the antibody or antigen binding portion thereof of the first and third aspects of the disclosure may be characterized by specific amino acid sequences in the regions determining its binding capability, such as the CDRs of the variable light and variable heavy chains, or indeed the entire variable light and/or heavy chain domains or regions. Non-limiting examples of such specific amino acid sequences are provided herein for the specific antibodies generated as described in Examples 9-12. It is contemplated that the specific sequence information provided for the generated antibodies enables the skilled person to define combinations and variations of these sequences within the scope of the invention.

Thus, in one embodiment, the antibody or antigen binding portion thereof comprises the following three CDRs in any combination of CDR-H1 / CDR-H2 / CDR-H3, e.g. in a heavy chain variable region when present:
- CDR-H1 selected from the group consisting of SEQ ID NO:10, 15, 18 and 21;
- CDR-H2 selected from the group consisting of SEQ ID NO:11, 13, 16, 19 and 22; and
- CDR-H3 selected from the group consisting of SEQ ID NO:12, 14, 17, 20 and 23.

In another embodiment, the antibody or antigen binding portion thereof comprises the following three CDRs in any combination of CDR-L1 / CDR-L2 / CDR-L3, e.g. in a light chain variable region when present:
- CDR-L1 selected from the group consisting of SEQ ID NO:24, 27, 29, 31 and 32;
- CDR-L2 being SEQ ID NO:25; and
- CDR-L3 selected from the group consisting of SEQ ID NO:26, 28, 30 and 33.

In another embodiment, the antibody or antigen binding portion thereof comprises the six CDRs CDR-H1 / CDR-H2 / CDR-H3 / CDR-L1 / CDR-L2 / CDR-L3 selected from the sequences listed above, in any combination thereof.

In specific embodiments of said first moiety, the combinations of CDRs are those present in the antibodies exemplified in Examples 9-12 (see Table 4).

In another more specific embodiment of the antibody or an antigen binding portion thereof according to the first or third aspect of the disclosure, the antibody or antigen binding portion thereof comprises a heavy chain variable region (VH) sequence selected from the group consisting of SEQ ID NO:34, 36, 38, 40, 42 and 43, and sequences having at least 70 % identity thereto.

In another more specific embodiment of the antibody or an antigen binding portion thereof according to the first or third aspect of the disclosure, the antibody or antigen binding portion thereof comprises a light chain variable region (VL) sequence selected from the group consisting of SEQ ID NO:35, 37, 39, 41 and 44, and sequences having at least 70 % identity thereto.

In one embodiment, the definitions of VH and VL sequences of the antibody or antigen binding portion thereof is limited to any one of the listed sequences and sequences having at least 75 %, such as at least 80 %, such as at least 85 %, such as at least 90 %, such as at least 95 %, such as at least 98 %, such as at least 100 % identity thereto.

In specific embodiments, the combinations of VH/VL are those present in the antibodies exemplified in Examples 9-12 (see Table 3 in particular).

For embodiments wherein the variable domains of the antibodies or antigen binding fragments are defined by a particular percentage sequence identity to a reference sequence, the VH and/or VL domains may retain identical CDR sequences to those present in the reference sequence such that the variation is present only within the framework regions.

As used herein, the terms "selective binding to X" and "affinity for X", wherein X is an antigen or an epitope, refer to a property of an antibody or antigen binding portion thereof which may be tested for example by ELISA, by surface plasmon resonance (SPR), by Kinetic Exclusion Assay (KinExA®) or by bio-layer interferometry (BLI). The skilled person is aware of these methods and others.

For example, binding affinity for antigen or epitope X may be tested in an experiment in which an antibody or antigen binding portion thereof to be tested is captured on ELISA plates coated with antigen X or an antigen exhibiting the epitope X, and a biotinylated detector antibody is added, followed by streptavidin-conjugated HRP. Alternatively, said detector antibody may be directly conjugated with HRP. TMB substrate is added and the absorbance at 450 nm is measured using an ELISA multi-well plate reader. The skilled person may then interpret the results obtained by such experiments to establish at least a qualitative measure of the binding affinity of the antibody or antigen binding portion thereof for X. If a quantitative measure is desired, for example to determine the EC50 value (the half maximal effective concentration) for the interaction, ELISA may also be used. The response of the antibody or antigen binding portion thereof against a dilution series of X may be measured using ELISA as described above. The skilled person may then interpret the results obtained by such experiments and EC50 values may be calculated from the results, using for example GraphPad Prism 5 and non-linear regression.

As used herein, the term "EC50" refers to the half maximal effective concentration of an antibody or antigen binding portion thereof which induces a response halfway between the baseline and maximum after a specified exposure time.

Additionally, inhibition ELISA may be used to obtain a quantitative measure of interaction by determination of the "IC50" (the half maximal inhibitory concentration). In an inhibition ELISA, the concentration of an antigen or epitope X in a fluid sample is measured by detecting interference in an expected signal output. In principle, a known antigen or epitope-bearing substance is used to coat a multiwell plate. In parallel, an antibody or antigen binding portion thereof with putative affinity for the antigen or epitope is added and incubated with a solution containing antigen at varied concentrations. Following standard blocking and washing steps, samples containing the mixture of said antibody or antigen binding portion thereof and the antigen or epitope are added to the well. Labeled detection antibody with affinity for the antigen- or epitope-binding antibody or antigen binding portion thereof is then applied for detection using relevant substrates (for example TMB). In principle, if there is a high concentration of antigen or epitope in the fluid sample, a significant reduction in signal output will be observed. In contrast, if there is very little antigen or epitope in the fluid sample, there will be very little reduction in the expected signal output. The skilled person appreciates that the signal output is also dependent on the affinity of the antibody or antigen binding portion thereof for said antigen or epitope.

As used herein, the term "IC50" refers to the half maximal inhibitory concentration of an antibody or antigen binding portion thereof which induces a response halfway between the baseline and maximum inhibition after a specified exposure time. Herein, a lower IC50 value indicates that a lower concentration of antigen or epitope is required to interfere with the binding of the detection antibody to the known antigen or epitope coated on the plate, as compared to a higher IC50 value. Thus, a lower IC50 value typically corresponds to a higher affinity.

The binding affinity of an antibody or antigen binding portion thereof may also be tested by surface plasmon resonance (SPR). For example, said binding affinity may be tested in an experiment in which antigen or epitope X is immobilized on a sensor chip of the instrument, and the sample containing the antibody or antigen binding portion thereof to be tested is passed over the chip. Alternatively, the antibody or antigen binding portion thereof to be tested may be immobilized on a sensor chip of the instrument, and a sample containing X is passed over the chip. The skilled person may then interpret the results obtained by such experiments to establish at least a qualitative measure of the binding affinity of the moiety for X. If a quantitative measure is desired, for example to determine a K_{D} value for the interaction, SPR may also be used. Binding values may for example be defined in a Biacore (GE Healthcare) or ProteOn XPR 36 (Bio-Rad) instrument. The antigen or epitope is suitably immobilized on a sensor chip of the instrument, and samples of the antibody or antigen binding portion thereof whose affinity is to be determined are prepared by serial dilution and injected. K_{D} values may then be calculated from the results using for example the 1:1 Langmuir binding model of the Biacore Insight Evaluation Software 2.0 or other suitable software, typically provided by the instrument manufacturer.

Another method for determining binding affinity of an antibody or antigen binding portion thereof to antigen or epitope X is the Kinetic Exclusion Assay (KinExA; Sapidyne Instruments Inc; Darling and Brault, Assay and Drug Dev Tech (2004) 2(6):647-657) for measurements of the equilibrium binding affinity and kinetics between unmodified molecules in solution. A KinExA K_{D} analysis requires immobilization of one interaction partner (e.g. the titrated binding partner) to a solid phase, which is then used as a probe to capture the other interaction partner (e.g. the constant binding partner) free in solution once an equilibrium is reached.

The binding affinity may also be measured by bio-layer interferometry (BLI), a label-free technology for measuring biomolecular interactions within the interactome. It is an optical analytical technique that analyzes the interference pattern of white light reflected from two surfaces: a layer of immobilized protein on the biosensor tip, and an internal reference layer. The binding between a ligand (antigen or epitope X) immobilized on the biosensor tip surface and an analyte (such as an antibody or antigen binding portion thereof with affinity for X) in solution produces an increase in optical thickness at the biosensor tip resulting in a wavelength shift, Δλ, which is a direct measure of the change in thickness of the biological layer. Interactions are measured in real time, providing the ability to monitor binding specificity, rates of association and dissociation, or concentration, with precision and accuracy.

The skilled person is aware of the above mentioned and other methods for measuring the affinity of an antibody or antigen binding portion thereof for antigen or epitope X, either qualitatively or quantitatively or both.

As used herein, the term "antibody or antigen binding portion thereof" encompasses not only full-length or intact polyclonal or monoclonal antibodies, but also antigen binding portions thereof, such as Fab, Fab', F(ab')₂, Fab₃, Fv and variants thereof, fusion proteins comprising one or more antibody portions, humanized antibodies, chimeric antibodies, minibodies, diabodies, triabodies, tetrabodies, linear antibodies, single chain antibodies, multispecific antibodies (e.g. bispecific antibodies) and any other modified configuration of the immunoglobulin molecule that comprises an antigen recognition site of the required specificity, including glycosylation variants of antibodies, amino acid sequence variants of antibodies and covalently modified antibodies. Further examples of modified antibodies and antigen binding portions thereof include nanobodies, AlbudAbs, DARTs (dual affinity re-targeting), BiTEs (bispecific T-cell engager), TandAbs (tandem diabodies), DAFs (dual acting Fab), two-in-one antibodies, SMIPs (small modular immunopharmaceuticals), FynomAbs (fynomers fused to antibodies), DVD-Igs (dual variable domain immunoglobulin), CovX-bodies (peptide modified antibodies), duobodies and triomAbs. This listing of variants of antibodies and antigen binding portions thereof is not to be seen as limiting, and the skilled person is aware of other suitable variants.

A full-length antibody comprises two heavy chains and two light chains. Each heavy chain contains a heavy chain variable region (VH) and first, second and third constant regions (CH1, CH2 and CH3). Each light chain contains a light chain variable region (VL) and a light chain constant region (CL). Depending on the amino acid sequence of the constant domain of its heavy chains, antibodies are assigned to different classes. There are six major classes of antibodies: IgA, IgD, IgE, IgG, IgM and IgY, and several of these may be further divided into subclasses, e.g., IgG1, IgG2, IgG3, IgG4, IgA1 and IgA2. The term "full-length antibody" as used herein, refers to an antibody of any class, such as IgD, IgE, IgG, IgA, IgM or IgY (or any sub-class thereof). The subunit structures and three-dimensional configurations of different classes of antibodies are well known.

The term "antigen binding portion" refers to a portion or region of an antibody molecule, or a derivative thereof, that retains all or a significant part of the antigen binding of the corresponding full-length antibody. An antigen binding portion may comprise the heavy chain variable region (VH), the light chain variable region (VL), or both. Each of the VH and VL regions or domains typically contains three complementarity determining regions CDR1, CDR2 and CDR3, denoted CDR-H1, CDR-H2 and CDR-H3 for the CDRs from the VH domain and CDR-L1, CDR-L2 and CDR-L3 for the CDRs from the VL domain. The three CDRs in VH or VL are flanked by framework regions (FR1, FR2, FR3 and FR4). As briefly listed above, examples of antigen binding portions include, but are not limited to: (1) a Fab fragment, which is a monovalent fragment having a VL-CL chain and a VH-CH1 chain; (2) a Fab' fragment, which is a Fab fragment with the heavy chain hinge region, (3) a F(ab')₂ fragment, which is a dimer of Fab' fragments joined by the heavy chain hinge region, for example linked by a disulfide bridge at the hinge region; (4) an Fc fragment; (5) an Fv fragment, which is the minimum antibody fragment having the VL and VH domains of a single arm of an antibody; (6) a single chain Fv (scFv) fragment, which is a single polypeptide chain in which the VH and VL domains of an scFv are linked by a peptide linker; (7) an (scFv)₂, which comprises two VH domains and two VL domains, which are associated through the two VH domains via disulfide bridges and (8) a domain antibody, which may be an antibody single variable domain (VH or VL) polypeptide that specifically bind antigen. Antigen binding portions can be prepared via routine methods. For example, F(ab')₂ fragments can be produced by pepsin digestion of a full-length antibody molecule, and Fab fragments can be generated by reducing the disulfide bridges of F(ab')₂ fragments. Alternatively, portions can be prepared via recombinant technology by expressing the heavy and light chain portions in suitable host cells (e.g., *E. coli,* yeast, mammalian, plant or insect cells) and having them assembled to form the desired antigen binding portions either *in vivo* or *in vitro.* A single-chain antibody can be prepared via recombinant technology by linking a nucleotide sequence coding for a heavy chain variable region and a nucleotide sequence coding for a light chain variable region. For example, a flexible linker may be incorporated between the two variable regions.

Furthermore, the skilled person is aware of the meaning of the terms polyclonal antibodies and monoclonal antibodies. Polyclonal antibodies are normally generated by administering an antigen to an animal. Said antigen will evoke an immune response giving rise to polyclonal antibodies. Monoclonal antibodies are made by immunizing an animal, usually a mouse, with an antigen and the subsequent isolation of the spleen from said animal. Isolated spleen cells are immortalized by fusion with myeloma cells to give rise to hybridoma cells. Each hybridoma cell produces a unique monoclonal antibody. The term "human antibody" as used herein, refers to antibodies having variable and constant regions corresponding to, or derived from, antibodies obtained from human subjects. The term "chimeric antibodies" as used herein, refers to recombinant or genetically engineered antibodies, such as for example antibodies with variable regions (VH and VL) of mouse origin and human constant region (Fc), to reduce the antibodies' immunogenicity. The term "humanized antibodies" refers to antibodies from non-human species whose protein sequences have been modified to increase their similarity to antibody variants produced naturally in humans, in order to reduce immunogenicity of the full antibody itself.

In yet another embodiment, the antibody or antigen binding portion thereof of the first and third aspects of the disclosure is selected from the group consisting of human antibodies, humanized antibodies and antigen binding portions thereof. In one particular embodiment, said antibody or antigen binding portion thereof is a humanized antibody or an antigen binding portion thereof.

### Pharmaceutical compositions

In a fourth aspect, there is provided a pharmaceutical composition comprising an antibody or antigen binding portion thereof as described herein and at least one pharmaceutically acceptable excipient or carrier.

Techniques for formulating antibodies for human therapeutic use are well known in the art and are reviewed, for example, in Wang et al., Journal of Pharmaceutical Sciences, Vol.96, pp1-26, 2007, the contents of which are incorporated herein in their entirety.

Pharmaceutically acceptable excipients that may be used to formulate the compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene- polyoxypropylene- block polymers, polyethylene glycol and wool fat.

In certain embodiments, the pharmaceutical compositions are formulated for administration to a subject via any suitable route of administration including but not limited to intramuscular, intravenous, intradermal, intraperitoneal injection, subcutaneous, epidural, nasal, oral, rectal, topical, inhalational, buccal (e.g., sublingual), and transdermal administration. In preferred embodiments, the composition is formulated for intravenous or subcutaneous administration.

### Methods of prevention, treatment, detection and diagnosis

The antibodies or antigen binding portions thereof according to the present disclosure may be useful as therapeutic and/or diagnostic agents.

Hence, in a fifth aspect of the present disclosure, there is provided an antibody or antigen binding portion thereof according to the first or third aspect, or a pharmaceutical composition according to the fourth aspect, for use as a medicament.

In a sixth aspect of the disclosure, there is provided an antibody or antigen binding portion thereof according to the first or third aspect, or a pharmaceutical composition according to the fourth aspect, for use as a diagnostic agent.

Also provided are methods of preventing, treating or diagnosing disease or assessing disease prognosis, wherein an antibody or antigen binding portion thereof as disclosed herein is administered to a subject, typically a human subject.

Also provided is the use of the disclosed antibodies or antigen binding portions thereof for the manufacture of compositions (such as medicaments) for use in the prevention, treatment, diagnosis and/or prognosis of any one of the listed diseases.

Also provided are methods of detecting or diagnosing a disease in a subject, wherein the methods comprise contacting a sample obtained from the subject with an antibody or antigen binding portion thereof as described herein. These methods are typically *in vitro* methods.

Thus, said antibody or antigen binding portion thereof, or pharmaceutical composition comprising it, is useful in the treatment, prevention and/or diagnosis of a condition selected from neurological disorders or conditions characterized by a loss of cognitive memory capacity. Such diseases or conditions include but are not limited to Alzheimer's disease (AD), mild cognitive impairment (MCI), dementia with Lewy body, Down's syndrome, hereditary cerebral hemorrhage with amyloidosis (Dutch type); as well as other diseases which are based on or associated with amylogenic proteins, such as cerebral amyloid angiopathy, Parkinson's disease, and cataract due to amyloid beta deposition.

Thus, in one embodiment, there is provided an antibody or antigen binding portion thereof, or pharmaceutical composition comprising it, for use in the treatment, prevention and/or diagnosis of an Aβ peptide-associated condition, such as amyloidosis. In one embodiment, there is provided an antibody or antigen binding portion thereof, or pharmaceutical composition comprising it, for use in the treatment, prevention and/or diagnosis of an Aβ peptide-associated condition, selected from the group consisting of Alzheimer's disease (AD), mild cognitive impairment (MCI), dementia with Lewy bodies, Down's syndrome, hereditary cerebral hemorrhage with amyloidosis (Dutch type), cerebral amyloid angiopathy, Parkinson's disease and cataract due to amyloid beta deposition. In one specific embodiment, said antibody or antigen binding portion thereof, or pharmaceutical composition comprising it, is provided for use in the treatment, prevention and/or diagnosis of Alzheimer's disease.

In a seventh aspect, there is provided a method of treatment, prevention and/or diagnosis of an Aβ peptide-associated condition in a mammal having, or being at risk of developing, said disorder, comprising administering to said mammal an amount, such as a therapeutically effective amount, of an antibody or antigen binding portion thereof, or pharmaceutical composition comprising it.

In one embodiment, said Aβ peptide-associated condition is, for example selected from the group consisting of amyloidosis, which refers to a group of diseases and disorders associated with amyloid plaque formation including secondary amyloidosis and age-related amyloidosis including, but not limited to, neurological disorders or conditions characterized by a loss of cognitive memory capacity such as, for example, Alzheimer's disease (AD), mild cognitive impairment (MCI), dementia with Lewy body, Down's syndrome, hereditary cerebral hemorrhage with amyloidosis (Dutch type); as well as other diseases which are based on or associated with amylogenic proteins, such as cerebral amyloid angiopathy, Parkinson's disease, and cataract due to amyloid beta deposition.

In a more specific embodiment, there is provided a method for treatment, prevention and/or diagnosis of an Aβ peptide-associated condition as disclosed herein, wherein said Aβ peptide-associated condition is selected from the group consisting of Alzheimer's disease (AD), mild cognitive impairment (MCI), dementia with Lewy bodies, Down's syndrome, hereditary cerebral hemorrhage with amyloidosis (Dutch type), cerebral amyloid angiopathy, Parkinson's disease and cataract due to amyloid beta deposition. In one particular embodiment, there is provided a method for treatment, prevention and/or diagnosis as disclosed herein, wherein said Aβ peptide-associated condition is Alzheimer's disease.

With regard to therapeutic or preventive use of the disclosed antibody, or antigen binding portion thereof, for the treatment of neurodegenerative diseases, there are several putative mechanisms of action. Without wishing to be bound by theory, non-limiting and independently possible mechanisms of action are for example i) the neutralization of putatively neurotoxic ApoE fragments and aggregated forms thereof; ii) Restoration of Aβ metabolism by removal of C-terminal ApoE fragments which have disrupted normal cholesterol/lipid transportation and thereby affected the processing of amyloid precursor protein (APP) so as to cause an increased production of Aβ; iii) reduction of Aβ aggregation caused by C-terminal ApoE fragments through binding of Aβ via the lipid binding region contained in these fragments; and iv) increased Aβ clearance in a situation wherein the C-terminal ApoE fragments form part of amyloid aggregates as such.

With regard to diagnostic or prognostic use of the disclosed antibody, or antigen binding portion thereof, in neurodegenerative diseases, the putatively neurotoxic ApoE fragments can be detected and measured in patients at risk of disease or showing signs of incipient disease. One such method is PET scan using a radio-labelled antibody of the disclosure. Another method for diagnosis and prognosis is biochemical analysis analyzing the levels of neurotoxic ApoE fragments in blood/plasma using ELISA/MSD.

While the invention has been described with reference to various exemplary aspects and embodiments, it will be understood by those skilled in the art that various changes may be made and equivalents may be substituted for elements thereof without departing from the scope of the invention. In addition, many modifications may be made to adapt a particular situation or molecule to the teachings of the invention without departing from the essential scope thereof. Therefore, it is intended that the invention not be limited to any particular embodiment, but that the invention will include all embodiments falling within the scope of the appended claims. The invention will be further illustrated by the following non-limiting Examples.

### Incorporation by Reference

Various publications are cited in the present application, each of which is incorporated by reference herein in its entirety.

### Examples

### Example 1

### Analysis of ApoE fragments in human brain extracts from Alzheimer's disease patients and controls

This example describes the homogenization of human brain tissues and the following Western blot analysis of ApoE fragments from brain extracts in Radio-Immunoprecipitation Assay (RIPA) buffer with 2% sodium dodecyl sulfate (SDS).

### Materials and methods

Brain tissue homogenization and sample preparation: Fresh frozen human brain tissue from Alzheimer's disease (AD) patients (n=24) and controls (n=14), with various APOE genotypes, were homogenized by 1:5 weight:volume in RIPA 2% SDS extraction buffer followed by a 16000 x *g* centrifugation for 1 h. The resulting supernatant was frozen at -80°C until analysis.

Analysis of ApoE fragments in human brain extracts: RIPA 2% SDS brain extract containing 10 µg total protein was mixed with 2x Laemmli sample buffer, boiled for 5 min at 95°C and loaded onto SDS-PAGE gels (Bolt™ 12% Bis-Tris Plus 10 well, Thermo Fisher). Gels were run for 30-40 min at 180 V, after which proteins were transferred from the gels to nitrocellulose membranes using the Trans-Blot® Turbo™ system (BioRad). Membranes were blocked in Odyssey® blocking buffer for 1 h and then incubated over night at room temperature with a polyclonal anti-ApoE antibody (Calbiochem, cat. No. #178479) diluted 1:2000 in Odyssey® blocking buffer with 0.1% Tween® 20. Membranes were washed and incubated for 1 h at room temperature with detection antibody anti-goat-800CW (LI-COR, cat. No 925-32214) diluted 1:25000 in Odyssey® blocking buffer with 0.1% Tween® 20. Membranes were washed and images acquired using Odyssey® FC (LI-COR). Image Studio Software (version 5.2) was used to quantify the relative amount of ApoE fragments in ratio to the amount of full-length ApoE in the acquired Western blot images.

### Results

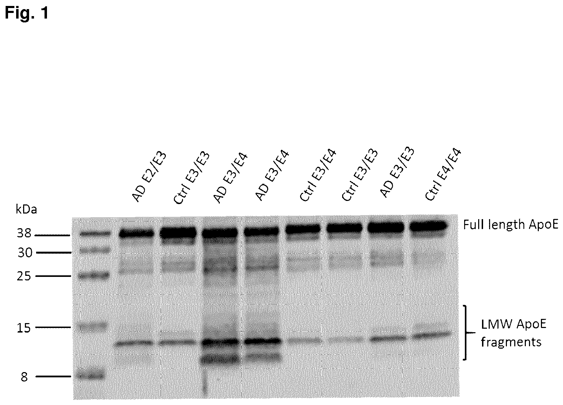

Full-length ApoE as well as several low molecular weight (LMW) ApoE fragments were identified by Western blot analysis of human brain RIPA 2% SDS extracts (n=38). Figure 1 shows a representative membrane from Western blot analysis. The LMW ApoE fragments were estimated to be 10, 12, 14-15 and 17 kDa in size (Figure 2).

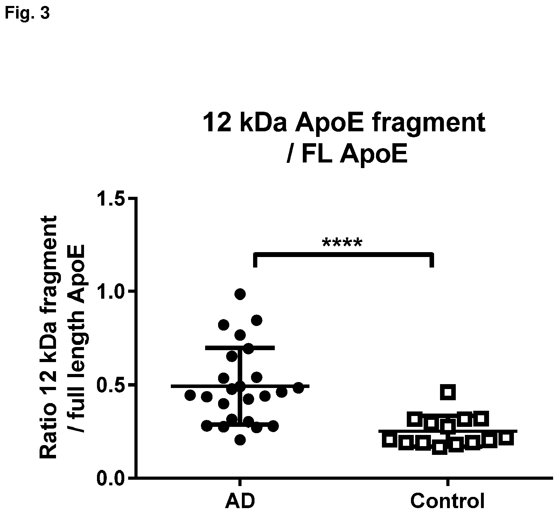

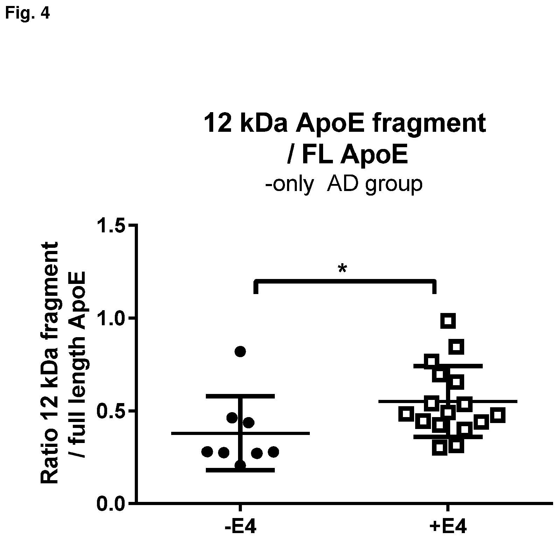

Analysis of ApoE fragments in ratio to full-length (FL) ApoE, demonstrated that the 12 kDa ApoE fragment was significantly increased in the AD group (n=24) as compared to the control group (n=14) (Figure 3). In addition, a significant increase of the 12 kDa ApoE fragment was observed in APOE ε4 carriers in the AD group (Figure 4).

### Example 2

### Extraction and isolation of ApoE fragments from human brain extracts from Alzheimer's disease patients

This example describes a procedure for isolation and concentration of full-length ApoE and 12 and 15 kDa ApoE fragments from human brain extracts, in order to prepare pure samples of ApoE with a protein concentration sufficient for amino acid sequence analysis.

### Materials and methods

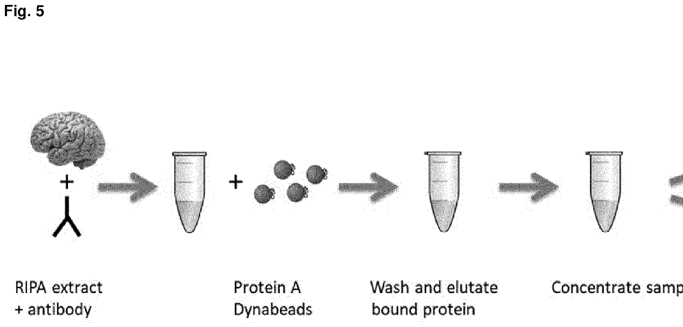

Isolation of ApoE from human brain extracts from AD patients with various APOE genotypes: A protocol for immunoprecipitation (IP) of ApoE from human brain extracts was established. Protocol optimization resulted in pure samples of ApoE with a protein concentration sufficient for amino acid sequence analysis. For a schematic overview of the workflow, see Figure 5. Human brain RIPA 2% SDS extracts, with a total protein content of 1.5 mg, were mixed with IP buffer (1xPBS, 0.05% Tween® 20, 0.1% Triton X-100, protease inhibitor cocktail) and ApoE was immunoprecipitated by adding 200 µg of an anti-ApoE C-terminal antibody, with a binding epitope within amino acids 237-299 (Thermo Scientific, cat. No PA5-27088). Complexes between IP antibody and ApoE in the brain extract were allowed to form during an incubation for 2 h at room temperature with head-over-tail rotation. 500 µl Protein A Dynabeads (Dynal, Thermo Scientific, cat. No 10002D) were added to the IP mixture and incubated for 1 h at room temperature with head-over-tail rotation, after which the Protein A Dynabeads were washed to remove unspecific binding to the beads. ApoE proteins bound to the Protein A Dynabeads (via the IP antibody) were eluted in 250 µl elution buffer (1.25 mM Tris pH 6.8, 0.005% SDS) and incubated for 5 min at 95°C with shaking at 900 rpm. After a quick spin, the samples were placed on the DynaMag™-2 magnet and the liquid was transferred to a new tube.

Concentration of isolated ApoE followed by analysis by SDS-PAGE: In order to concentrate the ApoE protein, the eluted IP sample was centrifuged in a rotational vacuum concentrator at 1300 rpm at 40°C for approximately 2 h, to reduce the volume from 250 µl to approximately 15 µl. 2x Laemmli buffer was added to the concentrated samples and the samples were incubated for 5 min at 95°C with 900 rpm. After a quick spin, the samples were loaded onto SDS-PAGE gels (Bolt™ 12% Bis-Tris Plus 10 well, Thermo Fisher, cat. No NW04120BOX). Gels were run for 30-40 min at 180 V, after which one gel was used for confirmation of ApoE fragments by Western blot analysis and one gel was silver stained and used for excision of ApoE.

Western blot analysis of SDS-PAGE gels: Proteins were transferred from the gels to nitrocellulose membranes using the Trans-Blot® Turbo™ system (BioRad). Membranes were blocked in Odyssey® blocking buffer for 1 h and then incubated over night at room temperature with the anti-ApoE C-terminal antibody (Thermo Scientific, cat. No PA5-27088) diluted 1:2000 in Odyssey® blocking buffer with 0.1% Tween® 20. Membranes were washed and incubated for 1 h at room temperature with detection antibody anti-rabbit-800CW (LI-COR, cat. No 925-32211) diluted 1:25000 in Odyssey® blocking buffer with 0.1% Tween® 20. Membranes were washed and images acquired using Odyssey® FC (LI-COR).

Silver staining of SDS-PAGE gels: Gels were fixated and stained with silver staining according to manufacturer's instructions (Pierce Silver Stain for Mass Spectrometry, Thermo Scientific, cat. No 24600). After the silver staining was complete, the stop buffer was exchanged to Milli-Q H₂O and rinsed 2x 10 min. Full-length ApoE, and the 12 and 15 kDa ApoE bands were excised from the gel and placed in Milli-Q H₂O in clean Eppendorf tubes.

### Results

Using the established IP protocol (Figure 5), ApoE was isolated from human AD brains with various APOE genotypes (ε2/ε3, ε3/ε3, ε3/ε4 and ε4/ε4), and the eluted proteins were run on SDS-PAGE.

Extraction of ApoE was confirmed by Western blot analysis. Figure 6 shows a representative Western blot membrane demonstrating several bands with ApoE fragments, as well as full-length ApoE. In addition, isolated and concentrated ApoE proteins were stained by silver staining of the SDS-PAGE gels as shown in Figure 7. ApoE fragments of approximately 12 and 15 kDa in size were visualized and excised from the silver stained gels. As reference samples, recombinant full-length ApoE protein and full-length ApoE from the human brain IP sample were also excised from the silver stained gels.

### Example 3

### Identification of trypsin cleavage sites in 12 kDa ApoE fragment

### Sample preparation

Silver-stained strips of gels from Example 2 in 1.5 ml PP-tubes, including a band of recombinant human full-length ApoE4 (rhApoE4) and/or 34 kDa from immunoprecipitation, band of 15 kDa from immunoprecipitation, and band of 12 kDa from immunoprecipitation, were washed with enough water and followed by dehydration using 500 µl acetonitrile (ACN; Wako). After turning each gel white, any solvent was removed and followed by adding 500 µl of water to get each gel swelling. After removal of water, 500 µl of Silver Quest Destainer (Invitrogen) was added to each gel and incubated for 15 min at room temperature. After removal of any destainer solvent, 1000 µl of water was added, then incubated for 10 min at room temperature. After the removal of water, 1000 µl of water was added again to wash each gel, then any solvent was removed from tubes. 500 µl ACN was added to each gel, then excess ACN was removed after turning each gel white.

500 µl of 10 mM dithiothreitol (DTT; Wako) was added into gels, followed by incubation at 56°C for 30 min. After removal of DTT solution, 500 µl ACN was added to shrink each gel with gentle mixing incubation at room temperature for 10 min. After removal of ACN, 55 mM iodoacetoamide (IAA; Wako) was added into each tube, then incubated at room temperature in the dark for 30 min. After removal of IAA solution, 500 µl ACN was added into each tube again, with occasional vortex mixing for 10 min, in order to obtain shrunk gels. After removal of ACN, 300 µl of 13 µg/ml trypsin in 10 mM ammonium bicarbonate with 10% ACN was added into the gels, then incubated at 5°C for 6 hours. Then, gels were placed in a 37°C chamber to promote digestion of proteins in each gel, followed by incubation overnight.

600 µl of 5% formic acid in water / ACN in a 1/2 (v/v) solution was added to each tube and mixed well with vortex. Then, incubation at 37°C with gentle rotating was conducted to obtain a solution including tryptic peptides from each gel. The obtained solution was dried by SpeedVac system (Thermo Fisher Scientific), followed by reconstitution using 300 µl of 5% methanol in 0.1% TFA-water. The solution was desalted by Monospin C18 solid extraction column (GL Sciences) according to the vendor's instruction manual, after which the eluent was dried by SpeedVac system. 30 µl of 5% methanol in 0.1% TFA-water was added into each tube to obtain the final reconstituted solution. The solution was subjected to LC-MS analysis.

### LC/MS analysis

The obtained samples were analyzed in a nano-flow LC-MS/MS system using a Q Exactive HF mass spectrometer (Thermo Fisher Scientific) coupled with an online UltiMate 3000 Rapid Separation LC (Dionex) and an HTC PAL sample injector (CTC Analytics) fitted with a microcapillary column (360 nm outer diameter (OD) × 100 µm ID), which was packed with < 20 cm of ReproSil C18-AQ 3 µm beads (Dr. Maisch GmbH) and equipped with an integrated electrospray emitter tip (P-2000 laser-based puller, Sutter Instruments). Each sample was loaded onto the capillary column by 4 µl full-loop mode injection. For LC separation, a mobile phase A of 4% ACN and 0.5% acetic acid (Wako) and a mobile phase B of 80% acetonitrile and 0.5% acetic acid were used for multiple linear gradient elution from 1-40% of B over 60 min, 40-70% of B over 10 min, and 70-99% of B over 5 min, and then held at 99% of B for 10 min at 500 nl/min. The total analysis time for each sample was 120 min.

Each sample was analyzed using data dependent analysis (DDA) mode, which used higher energy collision dissociation (HCD) MS/MS scans (resolution 30000) for the top 15 most abundant ions of each full-scan MS from m/z 300 to 3000 (resolution 60000) with a full-scan MS ion target of 3 × 10⁶ ions and an MS/MS ion target of 2 × 10⁵ ions. The maximum ion injection time for the MS/MS scans was 100 ms. The HCD normalized collision energy was set to 27, the dynamic exclusion time was set to 20 s, and the peptide match and isotope exclusion functions were enabled.

### Data analysis

All DDA mass spectra were analyzed with Proteome Discoverer ver. 2.1 (Thermo Fisher Scientific) using a human ApoE4 FASTA file (SEQ ID NO:45). SEQUEST-HT algorithm was used for MS/MS searching of the data sets with the following parameters: oxidation of methionine as variable modifications, carbamidomethylation of cysteine as a fixed modification, and trypsin as the digestion enzyme. Two missed cleavages per peptide were allowed. The mass tolerance for precursor ions was set to 10 ppm, and the mass tolerance for product ions was set to 20 mDa. A maximum false discovery rate (FDR) of 1% was applied for peptide identification. Protein identification required more than two peptides per protein. Then, a detailed analysis focusing only on ApoE4 was conducted to identify the cleavage sites of the 12 kDa band (ApoE4 fragment).

### Results

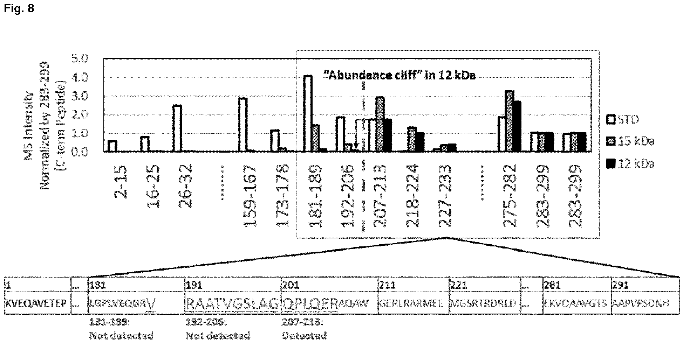

The 12 kDa ApoE fragment was subjected to tryptic digestion to survey the cleavage sites of ApoE on a peptide basis. rhApoE4 and 15 kDa bands were analyzed as references. The results (Figure 8) showed there was an "abundance cliff" in the tryptic peptides from the 12 kDa band between a peptide corresponding to amino acid residues 192-206 of ApoE and a peptide corresponding to amino acid residues 207-213. This means that there is at least one cleavage site in the region from amino acid residue 190 to amino acid residue 206, because the "207-213 peptide" was clearly detected with high MS intensity. Short peptides (less than 5 residues of amino acids) were eliminated from the analysis, so e.g. the VR dipeptide at positions 190-191 was not observed.

### Example 4

### Identification of LysC cleavage sites in 12 kDa ApoE fragment

### Materials and methods

Sample preparation, LC/MS analysis and data analysis were performed as described above for Example 3.

### Results

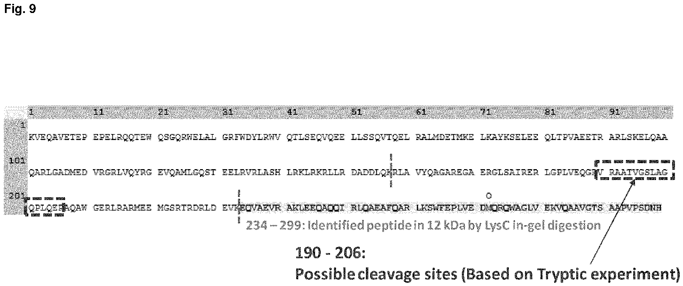

To narrow down the cleavage site of 12 kDa ApoE fragment on an amino acid basis, digestion by another enzyme, lysyl endopeptidase (LysC), was carried out. As a result of standard LysC proteomic analysis of the 12 kDa band (fixed cleavage at lysine C-terminal), the only peptide detected was a peptide corresponding to amino acid residues 234-299 of ApoE (Figure 9). This confirms the result of Example 3, to the effect that there is at least one cleavage site between positions 190-206. Notably, a peptide corresponding to amino acid residues 158-233 of ApoE was detected upon cleavage of rhApoE4 (not shown), but was not detected when cleaving the 12 kDa band, further supporting the existence of at least one cleavage site between positions 190-206.

### Example 5

### Further characterization of LysC cleavage sites in 12 kDa ApoE fragment

### Materials and methods

Sample preparation and LC/MS analysis were performed as described above for Example 4. Data analysis was performed as described above for Example 4, except that target analysis (describing peaks and the integration) from extracted-ion chromatograms (XIC) was performed for the specific peptides cleaved at unexpected regions. This peak qualification analysis was conducted by Qual Browser in Xcalibur 4.0 software (Thermo Fisher Scientific).

### Results

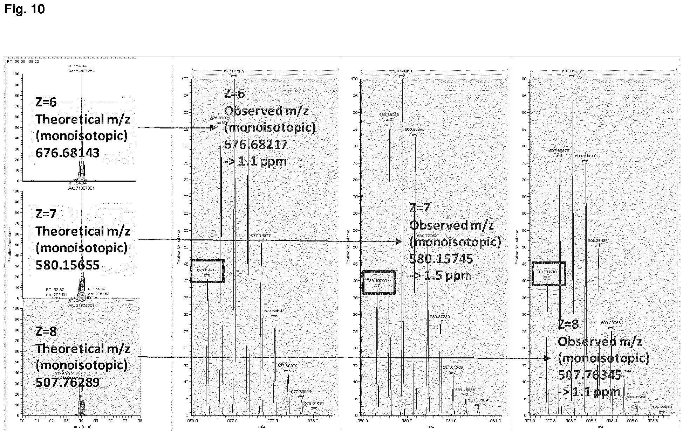

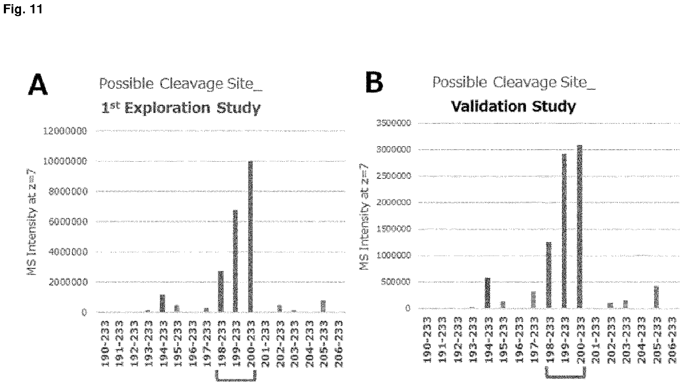

Prior to the detailed analysis of possible cleavage sites that give rise to the identified 12 kDa fragment, it was investigated whether the peptide corresponding to amino acid residues 158-233 of ApoE (SEQ ID NO:46) obtained by LysC digestion was detected in any of the rhApoE4 band, the 34 kDa band from immunoprecipitation, and the 12 kDa band from immunoprecipitation. This was done by describing each XIC with the theoretical m/z (z=10-15, 5 ppm mass tolerance). The results showed that the 158-233 peptide was clearly detected in the solution from rhApoE4 and the 34 kDa band, which means that there is no artifact cleavage in the sample preparation step. On the other hand, the 158-233 peptide was not observed in the sample solution from the 12 kDa band. That indicated that there is at least one cleavage site between aa 158 and aa 233 in the 12 kDa ApoE4 fragment. In summary, the LC/MS results from the tryptic process described in Example 3 elucidated the preliminary cleavage site between positions 190-205, then the site was confirmed by the LysC process as described in Example 4 and above. To narrow down the possible cleavage sites between 190-205 on an amino acid basis, all theoretical "non-conventional" peptides provided by LysC digestion of the 12 kDa band (i.e. 190-233, 191-233, 192-233, 193-233, 194-233, 195-233, 196-233, 197-233, 198-233, 199-233, 200-233, 201-233, 202-233, 203-233, 204-233, 205-233, and 206-233) were searched by describing each XIC to check whether the fragment peak was detected or not. Figure 10 shows an example of the results, when looking for "non-conventional LysC peptide" corresponding to amino acid residues 200-233 of ApoE (SEQ ID NO:47; [M]= 4054.04490). The theoretical monoisotopic m/z values (charges 6, 7 and 8) for the 200-233 peptide are 676.68143, 580.15655 and 507.76289, respectively. The extracted chromatogram for each m/z value provides a single peak at the same retention time, and the observed masses agree with the theoretical in each case with a mass accuracy of less than 2 ppm. These results strongly reinforced that non-conventional LysC peptides had been identified, leading to a positive identification of the specific cleavage sites that yield the 12 kDa ApoE fragment (Figure 11A). A duplicate experiment on another sample (ApoE e3/e4 allele) showed reproducible results (Figure 11B), confirming the determination of the cleavage sites.

In conclusion, nanoLC-MS/MS analysis of brain samples from three individual donors (ApoE ε3/ε4) demonstrated that the major cleavage sites that yield the 12 kDa ApoE fragment were at the N-terminus of L198, A199 and G200 (Figure 11).

### Example 6

### Identification of cleavage sites in 12 kDa ApoE fragment in human brains with ε4/ε4, ε2/ε3 and ε3/ε3 alleles

### Materials and methods

Sample preparation, LC/MS analysis and data analysis were performed as described above for Examples 3-5.

### Results

The N-termini L198, A199 and G200 were identified as the main cleavage sites to yield the 12 kDa ApoE fragment from ApoE ε3/ε4. To clarify if these cleavage sites are specific only to the ε4 allele and not to the ε2 or ε3 alleles, 12 kDa bands from the brains of ApoE ε4/ε4, ε2/ε3 and ε3/ε3 carriers were analyzed by means of the same manner as the previous section.

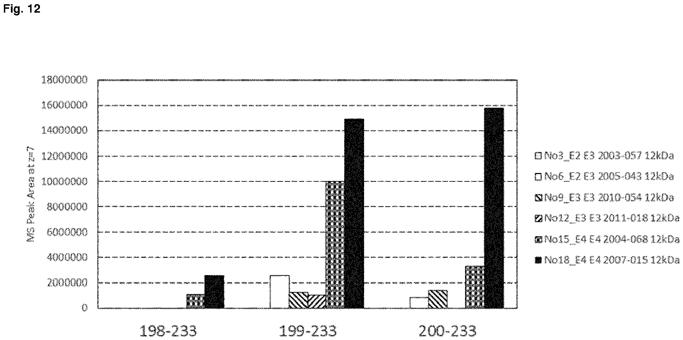

The results are presented in Figure 12 and showed that ε4/ε4 carriers exhibited the expected cleavages at the N-terminus of L198, A199 and G200 (mainly A199 and G200), whereas ε2/ε3 and ε3/ε3 carriers showed considerably lower signal of the sites cleavages than ε4/ε4 carriers. The results indicated that cleavage at the N-terminus of L198, A199 and G200 is more abundant in ε3/ε4 and ε4/e4 allele carriers.

### Example 7

### Neuronal toxicity of identified ApoE fragments

### Materials and methods

Cell culture: Neuro2A cells (ATCC) were seeded at 5.0 × 10⁴ cells/well in a 24 well plate (Falcon) and cultured in D-MEM High Glucose (WAKO) containing 10% fetal bovine serum. Transfection of pAAV-CMV vectors encoding human ApoE4 (full-length, SEQ ID NO:45) or the identified ApoE fragments (aa 198-299, SEQ ID NO:3; aa 199-299, SEQ ID NO:2; aa 200-299, SEQ ID NO:1) was done using Lipofectamine LTX and Plus Reagent (Invitrogen) on 1 day after seeding. 2 days later, vector-transfected cells were collected for Western blot analysis or seeded again at 2.0 × 10⁴ cells/well in a Seahorse XF96 cell culture microplate (Agilent Technologies) 4 hours before mitochondrial respiration measurement.

For assays using rat hippocampal neurons, the dissected hippocampi from fetuses obtained on embryonic day (E) 18 from timed pregnant Wistar rats (Charles River Laboratories) were digested using trypsinization and mechanical dissociation. The dissociated neurons were seeded at 1.5 × 10⁴ cells/well in Seahorse XF96 cell culture microplate (Agilent Technologies) for mitochondrial respiration measurement or 1.0 × 10⁵ cells/well in 24-well plate (Falcon) for Western blot analysis. Infection of AAV6 with full-length human ApoE4 or identified ApoE fragments (198-299, 199-299, 200-299) was performed at 7 days *in vitro* (DIV). Measurement of mitochondrial respiration or sample collection for Western blot analysis was performed at 7 days after infection (14 DIV).

Western blot analysis: Cells were lysed by RIPA buffer (50 mM Tris-HCI pH 7.6, 5 mM EDTA, 1 mM EGTA, 1% NP40, 0.25% sodium deoxycholate, 0.1 M NaCl, 0.5 mM PMSF) containing complete (EDTA-free) protease inhibitor cocktail (Roche) and PhosSTOP protein phosphatase inhibitor (Sigma), and sonicated. Sample Buffer Solution with Reducing Reagent (6x) (Nacalai Tesque) was added before SDS-PAGE. For SDS-PAGE, XV PANTERA MP Gel (DRC) 15% was used. For transfer, Trans-Blot Turbo (BIO-RAD) was used. For immunoblotting, iBind Western Systems (ThermoFisher Scientific) was used together with the following antibodies: anti-ApoE PA5-27088 (ThermoFisher Scientific); 178479 (Calbiochem).

Mitochondrial respiration measurement: Real-time measurement of oxygen consumption rates (OCR) was performed using an Extracellular Flux Analyzer XFe96 (Agilent Technologies). Before measurement, the culture medium was replaced by 37 °C pre-warmed XF Base Medium (Agilent Technologies) containing 10 mM sodium pyruvate (Sigma), 10 mM D-glucose (Sigma), 2 mM glutamine (Sigma). The pH of the measurement medium was adjusted to 7.4. The culture plates were incubated at 37 °C for 60 min prior to the assay. For analysis of mitochondrial function, XF Cell Mito Stress Test Kit (Agilent Technologies) was used. Following measurement of basal OCR, mitochondrial complex inhibitors were injected sequentially into each cell. The inhibitors were used at the following concentrations: oligomycin 1 µM; carbonyl cyanide 4-(trifluoromethoxy) phenylhydrazone (FCCP) 0.25 µM for Neuro2A cells, 2 µM for rat hippocampal neurons; rotenone/antimycin A 0.5 µM. OCR values were automatically calculated, recorded and plotted by the XFe96 software. Spare respiratory capacity was measured as (FCCP respiration - basal respiration).

### Results

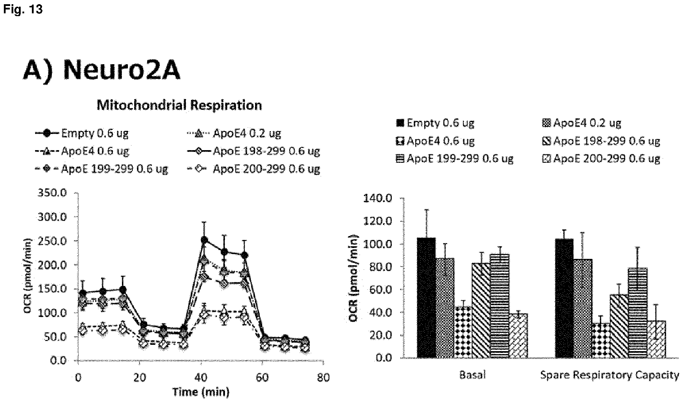

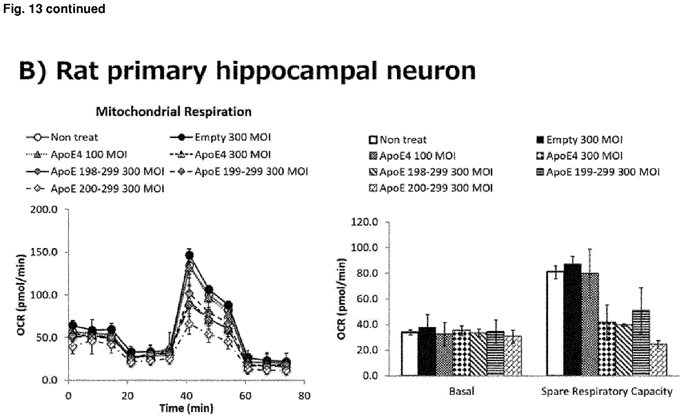

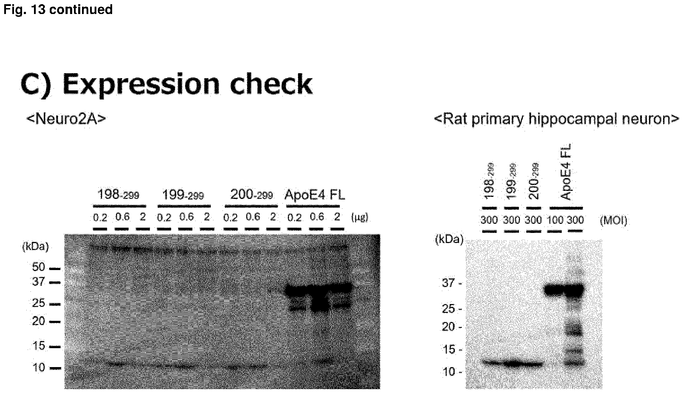

In both Neuro2A cells and rat primary hippocampal neurons, the groups expressing either one of the identified ApoE fragments (198-299, 199-299, 200-299) showed a reduction in spare respiratory capacity (Figure 13A and B), indicating that these fragments inflict mitochondrial damage. In addition, the fragments caused mitochondrial dysfunction at much lower expression levels than did full-length ApoE4 (Figure 13A-C). The results show that the C-terminal fragments of ApoE identified from human brain are neurotoxic.

### Example 8

### Plasmid preparation and antigen production

This example describes plasmid preparation and production by transient transfection of Expi293 cells and subsequent purification, to produce the antigens listed in Table 1 below.

**Table 1. Produced antigens**

| Antigen (short) | Info/Tags | Purification | SEQ ID NO: |
|---|---|---|---|
| G200-HIS | ApoE-G200-299H-10xHIS | IMAC, desalting | 48 |
| ApoE4-FL-HIS | 10xHIS-ApoE4(1-299) | IMAC, desalting | 49 |

Plasmid preparation: pcDNA3.4-TOPO plasmids with sub-cloned custom inserts of interest were ordered from GeneArt (Thermo Fisher Scientific). Plasmids were transformed into DH5α° *E*. *coli* to produce enough material for cell transfections, unless preparative amounts of plasmid DNA were ordered alongside the cloning services.

Transformations and plasmid preparations were performed using established protocols. Briefly, 50 µl of freshly thawed competent DH5α° *E. coli* were transformed by adding 10-100 ng plasmid, incubated for 30 min on ice, followed by a 45 s heat shock at 42 °C and a recovery on ice for 5 min, after which 250 µl SOC medium (Invitrogen) were added and the bacteria were cultured for 1 h at 37 °C. 25-100 µl of culture were used for streaking out on ampicillin-containing agar plates, from which colonies were selected the following day after incubation at 37 °C. Clones were selected from colony re-streaks.

Preparative cultures of LB medium with ampicillin were inoculated with clones and expanded at 37 °C overnight before pelleting the bacteria. Bacterial pellets were used to extract plasmid DNA using HiSpeed Maxi kits (Qiagen), and the supplier's instructions were followed throughout the procedure to obtain plasmid DNA.

Expression of antigen by transient transfection: The desired antigens were transiently expressed using the Expi293 system (Expi293 cells and ExpiFectamine™ 293 reagent; Thermo Fisher Scientific) and designed to be secreted into the supernatant.

Briefly, Expi293 cells were maintained in Expi293™ expression medium (37 °C, 8 % CO₂, 85 % humidity and 125 rpm). Cells seeded at 2 x 10⁶ /ml one day prior to transfection were transfected with 1 µg/ml of plasmid DNA complexes, prepared using the ExpiFectamine™ 293 reagent kit, as per the supplier's instructions (typically >95 % viable and counts 3-4 x 10⁶ /ml at Day 0). Enhancers I+II were added on Day 1 after transfection according to protocol. Cell viability was monitored regularly (AO/PI staining, Nexcelom K2 cellometer) and cultures harvested once viability dropped below 50 %. For harvesting, the supernatants were cleared by centrifugation (2000 g, 15 min, 4 °C), and then sterile filtered using 0.22 µm bottle top filters (Millipore). Filtered supernatant not immediately used for purification was stored at -80 °C for later processing.

Purification of HIS-tagged antigen by immobilized metal affinity chromatography (IMAC): The polyhistidine-tagged recombinant target proteins listed in Table 1 were purified by nickel-based IMAC, according to adapted protocols.

Briefly, input supernatants were re-filtered before being loaded onto pre-equilibrated (Buffer A: 20 mM Tris + 0.5 M NaCl, pH 8) IMAC columns (HisTrap Excel, GE Healthcare, cat. no. #17-3712-06). Unbound protein was washed out before applying elution gradients optimized for sample concentration, purity and collection (Buffer B: 20 mM Tris + 0.5 M NaCl + 500 mM imidazole, pH 8). Fractions were analyzed, and relevant ones pooled prior to buffer exchange.

Purified samples were buffer exchanged to sterile PBS (pH 7.4) using HiPrep™ 26/10 desalting columns (GE Healthcare, cat. no. #17-5087-01) and concentrated using Amicon® Ultra Centrifugal Filters (Millipore). As a standard, final products were sterile-filtered using 0.2 µm syringe filters (Pall).

### Example 9

### Generation and screening of antibodies to the N-terminal ApoE fragment neo-epitope G200

This example describes the immunization of Balb/c mice and subsequent generation and screening of hydridoma cell lines.

### Materials and methods

Peptide synthesis: The immunogen used in this experiment was designed to incorporate one of the N-terminal neo-epitopes of the neurotoxic ApoE fragment identified in the previous Examples. As its N-terminal sequence, the immunogen comprised the amino acid residues corresponding to amino acid residues 200-205 in full-length ApoE. This N-terminal sequence was coupled C-terminally to a 6-aminocaproic acid linker (Acp; also denoted aminohexanoic acid linker (Ahx)), followed by a cysteine residue for the purposes of conjugation to for example keyhole limpet hemocyanin (KLH) or bovine serum albumin (BSA) as indicated. The entire immunogen sequence used was GQPLQE-Acp-C (SEQ ID NO:50). SEQ ID NO:50 was prepared by Innovagen AB and delivered at 95.5 % purity.

Additionally, ApoE-derived peptides were prepared which incorporated the other two identified putative N-terminal neo-epitopes of the neurotoxic ApoE fragment, as well as a negative control peptide without any of the identified neo-epitopes. These peptides were likewise coupled to 6-aminocaproic acid and a cysteine residue. The first neo-epitope peptide comprised as its N-terminal sequence the amino acid residues corresponding to amino acid residues 199-204 in full-length ApoE. Thus, the entire sequence was AGQPLQ-Acp-C (SEQ ID NO:51). SEQ ID NO:51 was prepared by Innovagen AB and delivered at 96.7 % purity. The second neo-epitope peptide comprised as its N-terminal sequence the amino acid residues corresponding to amino acid residues 198-203 in full-length ApoE. Thus, the entire sequence was LAGQPL-Acp-C (SEQ ID NO:52). SEQ ID NO:52 was prepared by Innovagen AB and delivered at 95.2 % purity. The entire sequence of the negative control peptide was AATVGSLAGQPLQER-Acp-C (SEQ ID NO:53). SEQ ID NO:53 was prepared by Innovagen AB and delivered at 97.1 % purity.

Immunization: 10-12 weeks old Balb/c mice were immunized with SEQ ID NO:50 conjugated to KLH. In the first injection, the immunogen was given together with Freund's complete adjuvant. In subsequent injections except the last one, the immunogen was given in Freund's incomplete adjuvant. Plasma samples were collected three weeks after each immunization. Every mouse can receive up to 10 injections, but fewer immunizations were used for all of the mice. The final immunizations (booster dose) were administered intraperitoneally (i.p.) without adjuvant.

Plasma screening by direct ELISA: Plasma samples were analyzed by ELISA for reactivity against the target peptide SEQ ID NO:50 conjugated to bovine serum albumin, BSA, and against the recombinant ApoE fragment G200-HIS (SEQ ID NO:48) to determine when to stop immunizations and initiate hybridoma generation. Briefly, 96-well half area plates (Corning) were coated with 1 µg/ml antigen, i.e. G200 N-terminal neo-epitope peptide (ApoE sequence 200-205 incorporated in SEQ ID NO:50) coupled to BSA or purified recombinant C-terminal ApoE fragment G200-HIS (SEQ ID NO:48), in PBS at 50 µl/well over night at 4 °C. The plates were blocked with 150 µl/well of protein-free blocking solution (Pierce) for 1 h at room temperature with shake (600-900 rpm). The plates were washed four times with PBS containing 0.1 % TWEEN®-20. Plasma samples serially diluted with a starting dilution of 1/450 were added to the plates (dilution buffer: PBS with 0.1 % BSA and 0.05 % TWEEN®-20) and incubated for 2 h at room temperature. The plates were washed four times as before. Detection antibody (HRP-conjugated antimouse IgG, Southern Biotech, cat. no. 1030-05, diluted 1/10000 in dilution buffer) was added 50 µl/well, and the plates were incubated for 1 h at room temperature. After another wash (as above), 50 µl/well of K-Blue® Aqueous substrate (Neogen) was added, and the reaction was stopped after 10-15 min with 50 µl/well of 0.5-2 M H₂SO₄. The optical density at 450 nm was read using an ELISA reader (Tecan). The end point titers were defined as the dilution above the average of the blank wells (background) plus 3 standard deviations of the blank wells. An end point titer of 1/100,000 was considered high enough, and after this had been reached, no more immunizations were done until generation of hybridomas.

Generation of hybridomas: The mice received a final booster dose of immunogen i.p. (no adjuvant) 3 days before generation of hybridomas. Isolated splenocytes from sacrificed mice were fused with Sp2/0 cells at a 3:1 ratio and plated in 96-well plates in the presence of ClonaCell™-HY hybridoma selection medium D (StemCell Technologies), containing HAT for selection.

Wells were preferably screened twice during the next two weeks for reactivity to the G200-HIS recombinant ApoE fragment, and positive wells containing visible clones were processed further. Identified clones were expanded and subjected to at least two rounds of limiting dilution assay to assure monoclonality. Clones of interest were cryopreserved, expanded for production of antibody, and sequenced (Absolute Antibodies, UK). Secreted monoclonal antibodies were analyzed for isotype (Roche).

Hybridoma screening by direct ELISA: ELISA experiments to identify hybridoma clones producing antibodies with reactivity against the target epitope were performed according to standard ELISA protocols as described above in the section "Plasma screening by direct ELISA". During screening of hybridomas, and to reach monoclonality, three different antigens were used at a concentration of 1 µg/ml antigen. These were the G200 N-terminal neo-epitope peptide (ApoE sequence 200-205 incorporated in SEQ ID NO:50) coupled to BSA, purified recombinant C-terminal ApoE fragment G200-HIS (SEQ ID NO:48) and recombinant full-length ApoE4 (SEQ ID NO:45). Hybridoma supernatants were diluted 1:2 (dilution buffer: PBS with 0.1 % BSA and 0.05 % TWEEN®-20) and screened against binding to G200-HIS fragment. For the hybridoma screening ELISAs, "positive" wells were selected based on OD-values of >2 and the presence of clone/s. The identified positive clones were then subjected to positive and negative screenings using the same ELISA protocol, with G200-HIS fragment (SEQ ID NO:48), G200-peptide (SEQ ID NO:50) coupled to BSA, and ApoE4 full-length protein (SEQ ID NO:45) as coat for the plates. Clones that showed no substantial binding in the negative screening were subjected to 2 rounds of limiting dilution assay to ensure monoclonality and screened for binding to the G200-HIS fragment. Monoclonal clones that still bound G200-HIS fragment but not full-length ApoE4 and continued to grow were considered especially interesting for further characterization.

### Results

Generation of monoclonal antibodies by hybridoma technology: Antibodies that bind selectively to the N-terminal neo-epitope starting at amino acid G200 of the ApoE protein were generated by immunizations using an ApoE specific sequence consisting of the six first amino acids following the N-terminal in the 200-299 ApoE fragment. The shortness of the immunization peptide was considered necessary in order to enable generation of antibodies that bind selectively to the N-terminal neo-epitope starting at amino acid G200 of the ApoE protein, without any binding to the linear epitope found in full-length ApoE protein. The ApoE-specific sequence peptide 200-205, conjugated to keyhole limpet haemocyanin (KLH) via an Acp linker and a cysteine residue, was used in the immunizations. The plasma samples were analyzed by ELISA for reactivity against the corresponding peptide conjugated to BSA (to avoid detecting reactivity generated towards KLH), and against recombinant ApoE fragment. When titers were at least >1/100,000 the mice were sacrificed and the spleens were collected and used for hybridoma generation.

ELISA screening for antibodies that bind selectively to the N-terminal neo-epitope of ApoE fragment starting at G200: Generated hybridoma clones were screened for reactivity towards the recombinant ApoE fragment G200-HIS, as well as reactivity towards the target peptide SEQ ID NO:50 conjugated to BSA. In addition, lack of reactivity towards recombinant full-length ApoE4 was evaluated. Seven clones were identified as antibodies selective for the N-terminal neo-epitope of ApoE fragments starting at amino acid G200, and were denoted 4E6, 7B10, 7C7, 17G4, 21C3, 23D5 and 28F2.

### Example 10

### Characterization of monoclonal antibodies

This example describes the characterization, by direct ELISA, of monoclonal antibodies with affinity for the N-terminal neo-epitope of C-terminal ApoE fragments resulting from cleavage in the hinge region (starting at amino acid L198, A199 or G200). In addition, the monoclonal antibodies were evaluated for their ability to selectively bind to ApoE fragments (≤ 12 kDa) with the N-terminal neo-epitope starting at amino acid L198, A199 or G200 in Alzheimer's disease brain extracts, without any binding to full-length ApoE, using Western blot.

### Materials and methods

Selectivity evaluation by direct ELISA: The direct ELISA described below was used to evaluate the binding selectivity of generated monoclonal anti-ApoE antibodies with affinity for the N-terminal neo-epitope of C-terminal ApoE fragments starting at amino acid G200. The ability to selectively bind to N-terminal neo-epitope peptide (ApoE sequence aa 200-205, SEQ ID NO:6, incorporated in SEQ ID NO:50 for experimental purposes) and to the recombinant C-terminal ApoE fragment resulting from cleavage in the hinge region (ApoE sequence aa 200-299, SEQ ID NO:1, His-tagged for experimental purposes as SEQ ID NO:48) was compared with binding to recombinant full-length ApoE (ApoE4 sequence aa 1-299, SEQ ID NO:45).

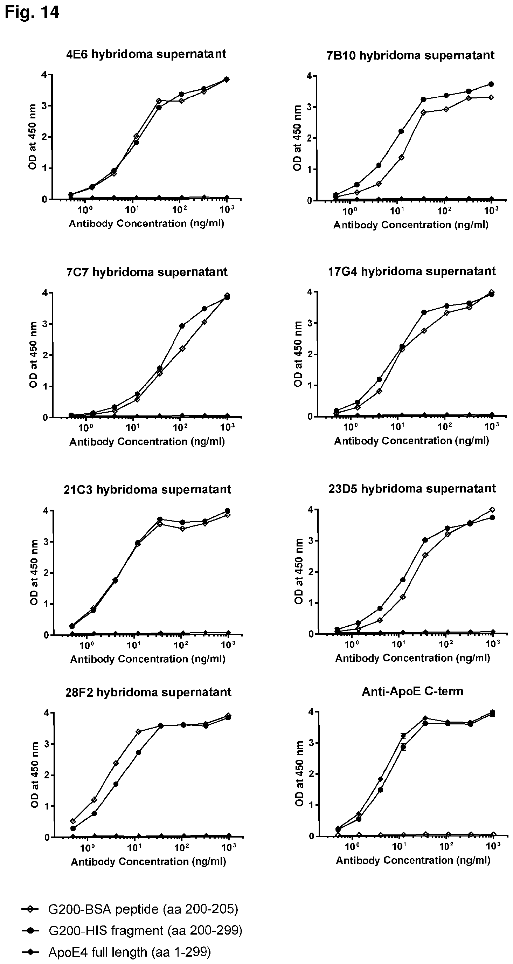

The screening was performed according to standard ELISA protocols. Briefly, 1 µg/ml solutions of N-terminal neo-epitope peptide (SEQ ID NO:50) conjugated to BSA, the recombinant C-terminal ApoE fragment G200-HIS (SEQ ID NO:48) and His-tagged full-length ApoE4 (SEQ ID NO:49) were prepared by dilution in PBS. 50 µl/well were added to an ELISA half-area 96 well microtiter plate. Then, the plate was sealed with adhesive sealer and incubated over night at 4 °C. The plates were blocked with 150 µl/well of protein-free blocking solution (Pierce) for 1 h at room temperature with shake (600-900 rpm). The plates were washed four times with PBS containing 0.1 % TWEEN®-20. Antibodies of interest were serially diluted with a starting dilution at 1 µg/ml (mixed 1:1 with dilution buffer: PBS with 0.1 % BSA and 0.05 % TWEEN®-20) and incubated for 2 h at room temperature. The plates were washed four times as before. Detection antibody (HRP-conjugated antimouse IgG, Southern Biotech, cat. no. 1030-05, diluted 1/10000 in dilution buffer) was added 50 µl/well, and the plates were incubated for 1 h at room temperature. After another wash (as above), 50 µl/well of K-Blue® aqueous substrate (Neogen) were added, and the reaction was stopped after 5-15 min with 50 µl/well of 0.5 M H₂SO₄. The optical density at 450 nm was read using an ELISA reader (Tecan). The optical density was plotted against the antibody concentration to generate concentration-response curves (Figure 14).

Isotyping: The isotype of each antibody clone was determined using Mouse Monoclonal Antibody Isotyping kit (Roche) according to the manufacturer's instructions.

Selectivity evaluation and human target binding in Alzheimer's disease brain extracts by Western blot analysis: The Western blot analysis described below was used to evaluate selective binding of monoclonal antibodies with affinity for the N-terminal neo-epitope of ApoE fragments ≤12 kDa, starting at G200, without binding to ApoE full-length protein, in human brain extracts from an Alzheimer's disease patient. Fresh frozen brain tissue from the Alzheimer's disease patient was homogenized in RIPA 2 % SDS extraction buffer, followed by centrifugation at 16000 x g for 1 h. The subsequent supernatant was subjected to standard protein concentration determination.

RIPA 2 % SDS brain extract containing 80 µg total protein was mixed with 2x Laemmli sample buffer, boiled for 5 min at 95 °C and loaded onto SDS-PAGE gels (Bolt™ 12 % Bis-Tris Plus 10 well, Thermo Fisher, cat. no. NW04120BOX). Gels were run for 30-40 min at 180 V, after which proteins were transferred from the gels to nitrocellulose membranes using the Trans-Blot® Turbo™ system (Bio-Rad). Membranes were blocked in Odyssey® blocking buffer for 1 h and incubated over night at room temperature with hybridoma supernatant diluted 1:1 in Odyssey® blocking buffer with 0.1 % TWEEN®-20. Membranes were washed and incubated for 1 h at room temperature with the detection antibody anti-mouse-800CW (LI-COR, cat. no. 925-32210) diluted 1:25000 in Odyssey® blocking buffer with 0.1 % TWEEN®-20. Membranes were washed and images acquired using Odyssey® FC (LI-COR).

To confirm that the obtained bands on the Western blot membranes were of ApoE origin, the membranes were re-stained over-night with a polyclonal anti-ApoE antibody (Calbiochem, cat. no. #178479; immunogen ApoE aa 1-299), diluted 1:2000 in Odyssey® blocking buffer with 0.1 % TWEEN®-20. Membranes were washed and incubated for 1 h at room temperature with detection antibody anti-goat-680RD (LI-COR, cat. no. 925-68074) diluted 1:25000 in Odyssey® blocking buffer with 0.1 % TWEEN®-20. Membranes were washed and images acquired using Odyssey® FC (LI-COR).

### Results

Selectivity evaluation: The seven hybridoma clones (4E6, 7B10, 7C7, 17G4, 21C3, 23D5 and 28F2) that were selective for the N-terminal neo-epitope of ApoE fragments starting at amino acid G200 demonstrated binding to the G200-BSA peptide (SEQ ID NO:50 conjugated to BSA) and to the recombinant G200-HIS fragment (SEQ ID NO:48), whereas no binding to recombinant ApoE4 full-length protein (aa 1-299; SEQ ID NO:45) was shown (Figure 14). The reference antibody ApoE Ab (Santa Cruz, cat. no. SC-393302, epitope ApoE aa 274-299) binds equally well to the recombinant G200-HIS fragment and to recombinant ApoE4 full-length protein. However, since the epitope for the reference antibody is ApoE aa 274-299, no binding to the G200-BSA peptide was shown, as expected. The experiment shown in Figure 14 was performed using unpurified cell supernatants from the monoclonal hybridomas 4E6, 7B10, 7C7, 17G4, 21C3, 23D5 and 28F2.

Isotyping: The isotype of the antibody clones, as determined using the Mouse Monoclonal Antibody Isotyping kit (Roche), is shown in Table 2.

**Table 2. Isotyping results**

| **Clone** | **Sub-class** | **Light chain** |
|---|---|---|
| 4E6 | IgG1 | κ |
| 7B10 | IgG1 | κ |
| 7C7 | IgG2b | κ |
| 17G4 | IgG1 | κ |
| 21C3 | IgG2b | κ |
| 23D5 | IgG1 | κ |
| 28F2 | IgG1 | κ |

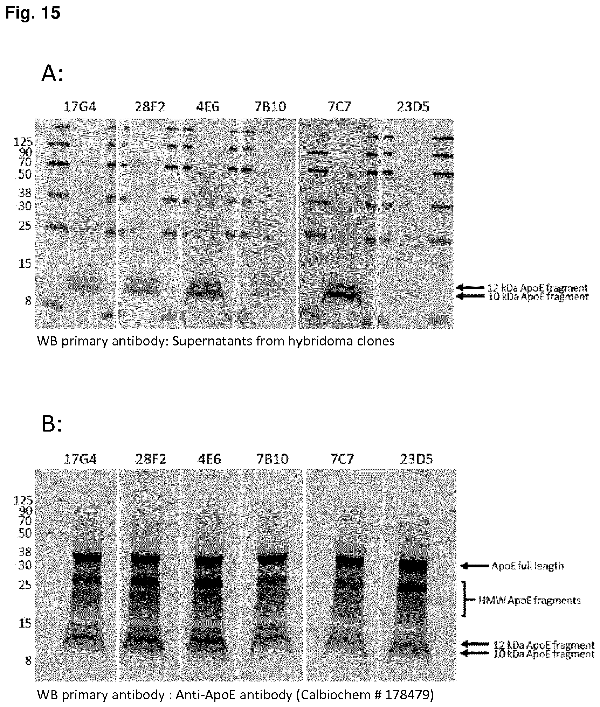

Selectivity evaluation and human target binding in Alzheimer's disease brain extracts by Western blot analysis: The hybridoma clones (4E6, 7B10, 7C7, 17G4, 21C3, 23D5 and 28F2) selective for the N-terminal neo-epitope of ApoE fragments starting at amino acid G200 were tested for their ability to bind selectively to ApoE fragments ≤12 kDa in human brain extracts from an Alzheimer's disease patient, and without any binding to full-length ApoE. Western blot analysis demonstrated binding of the monoclonal hybridomas to two ApoE fragments approximately 12 kDa and 10 kDa in size, without any visual binding to full-length ApoE (Figure 15A; data not shown for 21C3 because its sequence turned out to be identical to that of 4E6, see further below). Re-staining of the Western blot membranes with a polyclonal anti-ApoE antibody showed staining of full-length ApoE, high molecular weight (HMW) ApoE fragments (∼20-25 kDa) and a distinct 12 kDa ApoE fragment (Figure 15B). The 10 kDa ApoE fragment was less intensely stained when using the polyclonal anti-ApoE antibody as compared to using the monoclonal hybridomas. This reduced antibody binding to the 10 kDa fragment might be a result of C-terminal truncation in this fragment.

### Example 11

### Hybridoma sequencing and production of recombinant antibody

### Materials and methods

Hybridoma sequencing: Hybridoma clones producing monoclonal antibodies as generated and characterized in Examples 8-10, having a demonstrated selectivity for the N-terminal neo-epitope of ApoE fragment starting at amino acid G200, and a proven human target binding in brain extracts from Alzheimer's disease, were sent to Absolute Antibody for sequencing. Briefly, hybridoma sequencing was performed by whole transcriptome shotgun sequencing. The DNA and protein sequences of the mature VH and VL regions were identified.

Expression, production and purification of recombinant antibodies at Absolute Antibody: The variable domains were designed and optimized for expression in mammalian cells (HEK293) prior to being synthesized. The sequences were then subcloned into an Absolute Antibody cloning and expression vector for the appropriate isotype and subtype of immunoglobulin heavy and light chains.

HEK293 cells were passaged to the optimum stage for transient transfection. Cells were transiently transfected with heavy and light chain expression vectors and cultured for a further 6-14 days. An appropriate volume of cells were transfected with the aim of obtaining 2 mg of purified antibody.

Cultures were harvested and a one-step purification performed using affinity chromatography, after which the purified antibodies were buffer exchanged into PBS. Antibodies were analyzed for purity by SDS-PAGE and the concentration was determined by UV spectroscopy.

### Results

Hybridoma sequencing and recombinant antibody production: Hybridoma clones with a demonstrated selectivity for the N-terminal neo-epitope of ApoE fragment starting at amino acid G200, in addition to a demonstrated binding to human target in brain extracts from Alzheimer's disease, were sequenced.

The following hybridoma clones were sequenced: 4E6, 7B10, 7C7, 17G4, 21C3, 23D5 and 28F2. Sequencing revealed that antibodies 4E6 and 21C3 have the same sequence in both the primary VH and VL. The amino acid sequences of the entire antibodies were obtained. Amino acid sequences obtained for the respective variable heavy (VH) and variable light (VL) chains are given in Table 3 below.

**Table 3. Variable region sequences**

| Region | Antibody | SEQ ID NO: |
|---|---|---|
| | **4E6 / 21C3** | |
| VH | EVQLVESGGDLVKPGGSLKLSCAASGFTFSSYAMSWVRQSPEKRLE WVAEISGSGSRDHYTDSVTGRFTVSRDNAKNTLYLEMSSLRSEDTAIY YCARQLTGTDYYGTDYWGQGTSVTVSS | 34 |
| VL | DVLMTQIPLSLPVSLGDQASISCRSSQSIVYSNGNTYLEWYLQRPGQS PKLLIYKVSNRFSGVPDRFSGSGSGTDFTLKISRVEAEDLGVYYCFQG SHLPYTFGGGTKLEIK | 35 |
| | **7B10** | |
| VH | AVQLVESGGGLVKPGRSLKLSCAASGFTFSSYAMSWVRQFPDKRLD WVAEISSGGGSTNYLDTVTGRFTISRDNAKNTLYLEMNSLRSEDTAMY YCARQLVGTDYYGTDYWGQGTSVTVSS | 36 |
| VL | DVLMTQTPLSLPVSLGDQASISCRSSQNIVYSNGNTYLEWYLQKPGQS PKLLIYKVSNRFSGVPDRFSGSGSGTDFTLKISRVEAEDLGVYYCFQG SHVPYTFGGGTKLEIK | 37 |
| | **7C7** | |
| VH | EVQLVESGGGLVKPGGSLRLSCAASGFTFSSFAMSWVRQSPERRLE WVAEISRGGGYAFYSDTVTGRFTISRDNARNTLFLEMGSLRSEDTAMY YCSRQLTGTDYYAMDYWGQGTSVTVSS | 38 |
| VL | DVLLTQTPLSLFVSLGDQASISCRSSQSIVYTNGNTYLEWYLQKPGQS PKLLIYKVSNRFSGVPDRFSGSGSGTDFTLKISRVEAEDLGVYYCFQG SQVPYTFGGGTKLELK | 39 |
| | **17G4** | |
| VH | EVQLVESGGGLVKPGGSLKLSCSASGFTFSRYAMSWVRQSPEKRLE WVAEINSGGSYSFYSDTVTGRFTISRDNAKNTLFLEMSSLRSEDTAIYY CARQLTGTDYYGTDYWGQGTSVTVSS | 40 |
| VL | DVLMTQTPLSLPVSHGDQASISCRSSQSLLYSNGNTYLEWYLQKPGQ SPKLLIYKVSNRFSGVPDRFSGSGSGTDFTLKISRVEAEDLGVYYCFQ GSHVPYTFGGGTKLEIK | 41 |
| | **23D5** | |
| VH | EVQLVESGGGLVKPGGSLKLSCSASGFTFSRYAMSWVRQSPEKRLE WVAEINSGGSYSFYSDTVTGRFTISRDNAKNTLFLEMSSLRSEDTAIYY CARQLSGTDYYGTDYWGQGTSVTVSS | 42 |
| VL | DVLMTQTPLSLPVSHGDQASISCRSSQSLLYSNGNTYLEWYLQKPGQ SPKLLIYKVSNRFSGVPDRFSGSGSGTDFTLKISRVEAEDLGVYYCFQ GSHVPYTFGGGTKLEIK | 41 |
| | **28F2** | |
| VH | QIQLVQSGPDLKKPGETVKISCKASGYTFTNYAMHWMKQAPGKALKW MGWINTYTGEPTFADDFKGRFAFSLETSASTAYLQINSLKNEDMATYF CAREGYYDRSHYFDYWGQGTTLTVSS | 43 |
| VL | DVLMTQTPLSLPVSLGDQASISCRSSLSLVHGDGNTYLEWYLQKPGQ SPKLLIYKVSNRFSGVPDRFSGSGSGTDFTLKISRVEAEDLGVYYCLQ GSHIPFTFGSGTKLEIK | 44 |

The complementarity determining regions (CDRs) were identified using the Kabat definition for the primary VH and VL sequences and are given in Table 4 below.

**Table 4. CDR region sequences**

| **Antibody** | **CDR H1** | **CDR H2** | **CDR H3** |
|---|---|---|---|
| 4E6/21C3 | SYAMS (SEQ ID NO:10) | EISGSGSRDHYTDSVTG (SEQ ID NO:11) | QLTGTDYYGTDY (SEQ ID NO:12) |
| 7B10 | SYAMS (SEQ ID NO:10) | EISSGGGSTNYLDTVTG (SEQ ID NO:13) | QLVGTDYYGTDY (SEQ ID NO:14) |
| 7C7 | SFAMS (SEQ ID NO:15) | EISRGGGYAFYSDTVTG (SEQ ID NO:16) | QLTGTDYYAMDY (SEQ ID NO:17) |
| 17G4 | RYAMS (SEQ ID NO:18) | EINSGGSYSFYSDTVTG (SEQ ID NO:19) | QLTGTDYYGTDY (SEQ ID NO:12) |
| 23D5 | RYAMS (SEQ ID NO:18) | EINSGGSYSFYSDTVTG (SEQ ID NO:19) | QLSGTDYYGTDY (SEQ ID NO:20) |
| 28F2 | NYAMH (SEQ ID NO:21) | WINTYTGEPTFADDFKG (SEQ ID NO:22) | EGYYDRSHYFDY (SEQ ID NO:23) |

| | **CDR L1** | **CDR L2** | **CDR L3** |
|---|---|---|---|
| 4E6/21C3 | RSSQSIVYSNGNTYLE (SEQ ID NO:24) | KVSNRFS (SEQ ID NO:25) | FQGSHLPYT (SEQ ID NO:26) |
| 7B10 | RSSQNIVYSNGNTYLE (SEQ ID NO:27) | KVSNRFS (SEQ ID NO:25) | FQGSHVPYT (SEQ ID NO:28) |
| 7C7 | RSSQSIVYTNG NTYLE (SEQ ID NO:29) | KVSNRFS (SEQ ID NO:25) | FQGSQVPYT (SEQ ID NO:30) |
| 17G4 | RSSQSLLYSNGNTYLE (SEQ ID NO:31) | KVSNRFS (SEQ ID NO:25) | FQGSHVPYT (SEQ ID NO:28) |
| 23D5 | RSSQSLLYSNGNTYLE (SEQ ID NO:31) | KVSNRFS (SEQ ID NO:25) | FQGSHVPYT (SEQ ID NO:28) |
| 28F2 | RSSLSLVHGDGNTYLE (SEQ ID NO:32) | KVSNRFS (SEQ ID NO:25) | LQGSHIPFT (SEQ ID NO:33) |

The monoclonal antibodies 4E6, 7B10, 7C7, 17G4, 23D5 and 28F2 were selected for production as recombinant IgG2c antibodies, whereas 21C3 was not produced (because of the sequence redundancy with 4E6). All recombinant antibodies were successfully produced and purified to a final concentration of 1 mg/ml. Antibody purity, as defined by SDS-PAGE, was >98 % for all antibodies.

### Example 12

### Characterization of recombinant antibodies

This example describes the characterization of the recombinant antibodies produced in Example 11 by various methods, including direct ELISA, inhibition ELISA and biolayer interferometry.

### Materials and methods

Selectivity evaluation of recombinant antibodies by direct ELISA: The direct ELISA described below was used to evaluate the binding selectivity of the recombinant antibodies produced in Example 11. The ability to selectively bind to the G200 N-terminal neo-epitope ApoE peptide (BSA-conjugated SEQ ID NO:50) and to the recombinant G200-HIS C-terminal ApoE fragment resulting from cleavage in the hinge region (SEQ ID NO:48) was compared with binding to the L198, A199 and negative control peptides (BSA-conjugated SEQ ID NO:51, 52 and 53, respectively), and to His-tagged, recombinant full-length ApoE4 (ApoE sequence aa 1-299; SEQ ID NO:49).

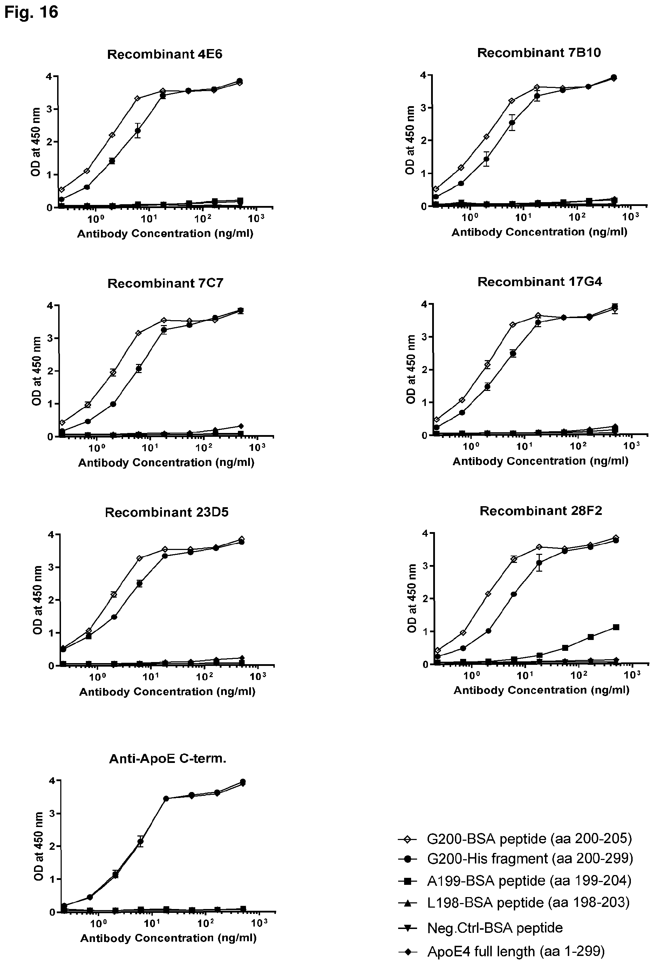

The screening was performed according to standard ELISA protocols. Briefly, 1 µg/ml solutions of BSA-conjugated neo-epitope peptides, recombinant C-terminal ApoE fragment, negative control peptide and full-length ApoE were prepared by dilution in PBS. 50 µl/well were added to an ELISA half-area 96 well microtiter plate, the plate was sealed with adhesive sealer and incubated overnight at 4 °C. After discarding the solution, the plates were blocked with 150 µl/well of protein-free blocking solution (Pierce) for 1 h at room temperature with shake (900 rpm). The plates were washed four times with PBS containing 0.1 % TWEEN®-20. Generated recombinant antibodies of interest were serially diluted in 1:3 ratio with a starting dilution at 0.5 µg/ml (mixed 1:1 with dilution buffer: PBS with 0.1 % BSA and 0.05 % TWEEN®-20). An anti-ApoE C-terminal antibody was used as a reference antibody (ApoE E-8 mouse monoclonal, cat. no. sc-393302; Santa Cruz Biotechnology). 50 µl/well were added into the ELISA plates and incubated for 2 h at room temperature with shake (900 rpm). The plates were washed four times as described previously. Detection antibody (HRP-conjugated anti-mouse IgG, Southern Biotech, cat. no. 1030-05, diluted 1:10000 in dilution buffer) was added 50 µl/well, and the plates were incubated for 1 h at room temperature with shake (900 rpm). After another wash (as described previously), 50 µl/well of K-Blue® Aqueous substrate (Neogen) were added, and the reaction was stopped after 5-15 min with 50 µl/well of 0.5 M H₂SO₄. The optical density at 450 nm was read using an ELISA reader (Tecan). The optical density was plotted against the antibody concentration to generate concentration-response curves (Figure 16).

Selectivity evaluation and IC50 determination of recombinant antibodies by inhibition ELISA: The inhibition ELISA described below was used to evaluate the binding strength and selectivity for the recombinant antibodies produced in Example 11. The ability of the recombinant antibodies to bind to the N-terminal neo-epitope of synthetic ApoE peptides starting at amino acid G200 and to recombinant C-terminal ApoE fragment G200-HIS was evaluated in comparison with their ability to bind to synthetic ApoE peptides starting at amino acids L198 and A199, as well as to full-length ApoE4 (aa 1-299) in solution.

In brief, the recombinant antibody to be tested was allowed to interact with the N-terminal neo-epitope of synthetic ApoE peptides conjugated to BSA and starting at amino acid L198 (SEQ ID NO:52), A199 (SEQ ID NO:51) or G200 (SEQ ID NO:50), or to BSA-conjugated negative control peptide (SEQ ID NO:53), or to His-tagged recombinant C-terminal ApoE fragment G200-HIS (SEQ ID NO:48), or to His-tagged recombinant full-length ApoE4 in solution (SEQ ID NO:49). Thereafter, the mix is added to a microtiter plate coated with the BSA-coupled G200 synthetic ApoE peptide. If the recombinant antibody binds to any of the antigens in the pre-incubation step (the synthetic ApoE peptides, recombinant C-terminal ApoE fragment or full-length ApoE4), the antibody is prevented from binding to the synthetic G200 ApoE peptide immobilized on the microtiter plate. This leads to inhibition of the ELISA detection signal.

0.5 µg/ml solution of N-terminal neo-epitope peptide G200 (SEQ ID NO:50) conjugated to BSA was prepared by dilution in PBS. 50 µl/well were added to an ELISA half-area 96 well microtiter plate, the plate was sealed with adhesive sealer and incubated overnight at 4 °C. After discarding the above solution, the plates were blocked with PBS-Tween 20 (0.05 %) (150 µl/well) at room temperature for at least 1 h with shaking (900 rpm). Serial dilutions of each synthetic ApoE peptide, recombinant C-terminal ApoE fragment G200-HIS and full-length ApoE4 were prepared in a 1:3 ratio starting from 1000 ng/ml in 96 well storage plates (30 µl final volume). Each recombinant antibody to be tested was added (30 µl) to the serial dilutions of each of the synthetic ApoE peptides, G200-HIS or full-length ApoE4at a final concentration of 0.1 µg/ml per well. Samples were pre-incubated for 45 min at room temperature with shaking (900 rpm). The plate coated with the N-terminal neo-epitope peptide G200 was washed four times with PBS containing 0.05% TWEEN®-20, and 0.1% BSA.

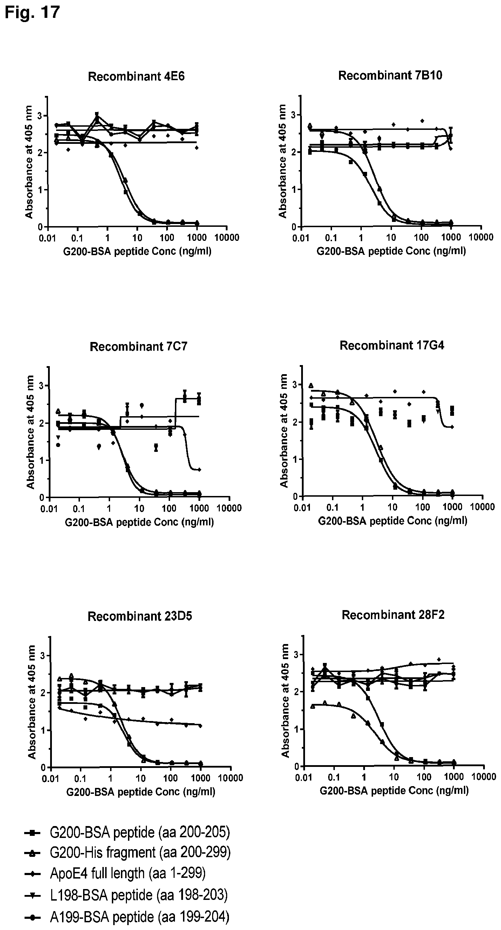

The pre-incubated samples were transferred (50 µl/well) to the blocked ELISA plates, and plates were incubated for 25 min at room temperature without shaking. Plates were washed as described above. Alkaline phosphatase conjugated anti-mouse IgG detection antibody (Mabtech, cat. no. 3310-4) was diluted 1:1000 and added to each plate (50 µl/well). The plate was sealed and incubated with shaking (900 rpm) for 45 min at room temperature and subsequently washed as described above. Alkaline phosphatase substrate (50 µl/well) was added to the plate and the optical density was read every 15 min at a wavelength of 405 nm for up to 120 min. The IC50 values were determined from a log inhibitor concentration response curve (Figure 17).

Selectivity evaluation and K_{D} determination of recombinant antibodies by bio-layer interferometry: Binding interactions between the N-terminal neo-epitope peptide G200 (SEQ ID NO:50) conjugated to BSA (ligand) and the recombinant antibodies produced in Example 11 (analyte) were evaluated using an Octet RED384 instrument (ForteBio). All analyzed proteins were diluted in 1x Kinetics Buffer (ForteBio).

To analyze the antibody-target interaction and to determine the binding affinity of the recombinant antibodies for recombinant C-terminal ApoE fragment G200-HIS (SEQ ID NO:48), anti-HIS capture biosensors (HIS1K) were used. In the first step (loading step), HIS-tagged recombinant C-terminal ApoE fragment was captured onto the surface of the biosensor. Next, a concentration gradient of pure antibodies was prepared ranging from 150 nM to 2.5 nM in 2-fold serial dilutions. Subsequently, the HIS1K biosensors with ligand were dipped in wells containing diluted antibodies, and the association phase was monitored for 600 s. For monitoring the dissociation phase, the HIS1K biosensors with ligand were moved to a well containing 1x Kinetics Buffer and the dissociation was monitored for 1000 s. For evaluation of the kinetic experiments, the Octet Data Analysis software was used. All values collected from interaction analysis were blank subtracted, and a 1:2 binding kinetics global fit model (bivalent analyte) was used for the evaluation.

### Results

Selectivity evaluation of recombinant antibodies by direct ELISA: The six produced recombinant antibodies 4E6, 7B10, 7C7, 17G4, 23D5 and 28F2 were screened for reactivity towards the BSA-conjugated peptides 198-203, 199-204 or 200-205 and the ApoE recombinant fragment aa 200-299. In addition, absence of reactivity towards recombinant full-length ApoE (ApoE4 1-299) and the negative control peptide (SEQ ID NO:53) conjugated to BSA was studied.

All recombinant antibodies demonstrated strong binding to the N-terminal neo-epitope peptide G200 (SEQ ID NO:50) and to the recombinant C-terminal ApoE fragment G200-HIS (aa 200-299; SEQ ID NO:48), whereas no binding to recombinant ApoE4 full-length protein (SEQ ID NO:49) or to the negative control peptide (SEQ ID NO:53) was shown (Figure 16). In addition, no binding to the 198-203 or 199-204 BSA-conjugated peptides (SEQ ID NOs:51 and 52) were observed using the recombinant anti-G200 antibodies. On the other hand, the reference antibody directed to the ApoE C-terminal could detect and bind the recombinant G200 fragment (aa 200-299) and the recombinant ApoE4 full-length protein (aa 1-299) equally well. Because the epitope for the reference antibody is ApoE aa 274-299, no binding to the G200-BSA peptide was shown, as expected (Figure 16).

Selectivity evaluation and IC50 determination of recombinant antibodies by inhibition ELISA: The six produced recombinant antibodies 4E6, 7B10, 7C7, 17G4, 23D5 and 28F2 were screened for binding strength and binding selectivity towards the N-terminal neo-epitope peptide G200-BSA (aa 200-205) and the recombinant C-terminal ApoE fragment G200-HIS (aa 200-299). In addition, antibodies were screened for lack of reactivity towards BSA-conjugated peptides 198-203 and 199-204, and recombinant full-length ApoE4.

All recombinant antibodies demonstrated strong binding and selectivity for the G200-BSA peptide (aa 200-205) and the recombinant G200 fragment (aa 200-299), whereas no binding to recombinant ApoE4 full-length protein (aa 1-299) was shown (Figure 17). In addition, no binding was shown to 198-203 or 199-204 BSA-conjugated peptides. The calculated IC50 values (ng/ml and nM values) are shown in Table 5 below.

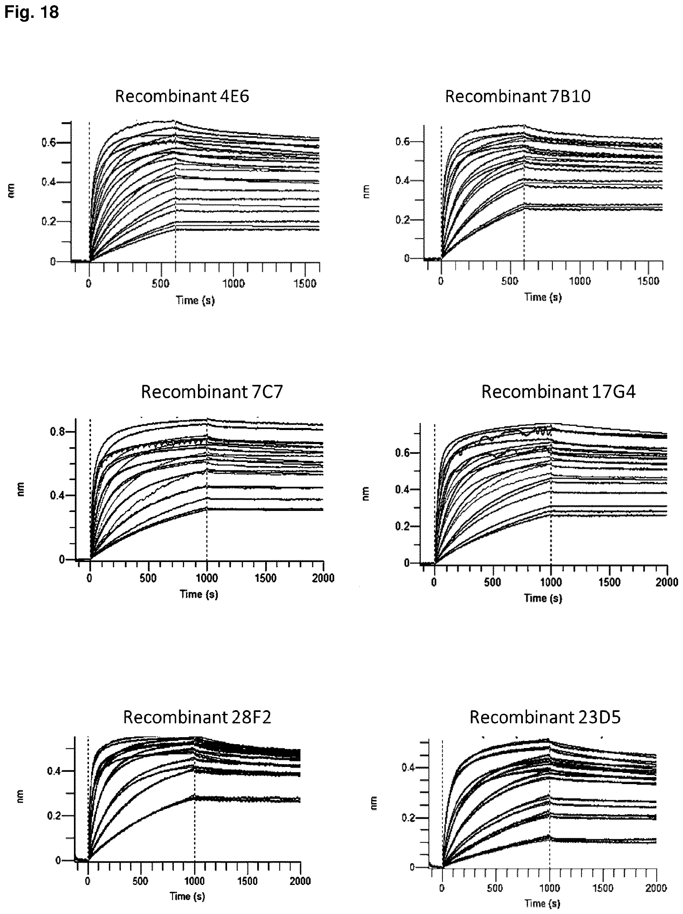

Selectivity evaluation and K_{D} determination of recombinant antibodies by bio-layer interferometry: Binding interactions between the recombinant G200 C-terminal ApoE fragment (ligand) and recombinant antibodies (analyte) were evaluated by using an Octet RED384 instrument (ForteBio). The results from the Octet experiment are outlined in Figure 18 and summarized in Table 5 below.

**Table 5. Summary of results from inhibition ELISA and biolayer interferometry analysis of recombinant anti-G200 antibodies**

| Clone | IC50 (ng/ml) G200 BSA-peptide | IC50 (ng/ml) G200-HIS fragment | K_{D} (nM) G200-HIS fragment on biosensor |
|---|---|---|---|
| 4E6 | 2.88 | 3.97 | 0.9 |
| 7B10 | 2.18 | 2.84 | 0.8 |
| 7C7 | 3.10 | 2.83 | 1.9 |
| 17G4 | 2.92 | 3.02 | 0.8 |
| 23D5 | 2.77 | 2.35 | 5.2 |
| 28F2 | 3.17 | 2.37 | 9.7 |

### Example 13

### Generation and screening of antibodies to the N-terminal ApoE fragment neo-epitope L198

In this Example, mice are immunized, hybridomas screened and monoclonal antibodies identified essentially as described in Example 9, with the exception that the immunogen used is LAGQPLQE-Acp-C (SEQ ID NO:54) conjugated to KLH. The identified monoclonal antibodies are characterized essentially as described in Example 10, and are expected to show selective binding to the neo-epitope on the 12 kDa ApoE fragment characterized by the N-terminal L198 residue. Further investigation is performed as described in Example 11-12.

### Example 14

### Generation and screening of antibodies to the N-terminal ApoE fragment neo-epitope A199

In this Example, mice are immunized, hybridomas screened and monoclonal antibodies identified essentially as described in Example 9, with the exception that the immunogen used is AGQPLQE-Acp-C (SEQ ID NO:55) conjugated to KLH. The identified monoclonal antibodies are characterized essentially as described in Example 10, and are expected to show selective binding to the neo-epitope on the 12 kDa ApoE fragment characterized by the N-terminal A199 residue. Further investigation is performed as described in Example 11-12.

### Sequence Listing

Table 6 below lists the sequences referred to in this application.

| **SEQ ID NO:** | **Description/occurrence** | **Sequence** |
|---|---|---|
| 1 | ApoE 200-299 | GQPLQERAQAWGERLRARMEEMGSRTRDRLDEVKE QVAEVRAKLEEQAQQIRLQAEAFQARLKSWFEPLVE DMQRQWAGLVEKVQAAVGTSAAPVPSDNH |
| 2 | ApoE 199-299 | AGQPLQERAQAWGERLRARMEEMGSRTRDRLDEVK EQVAEVRAKLEEQAQQIRLQAEAFQARLKSWFEPLVE DMQRQWAGLVEKVQAAVGTSAAPVPSDNH |
| 3 | ApoE 198-299 | LAGQPLQERAQAWGERLRARMEEMGSRTRDRLDEV KEQVAEVRAKLEEQAQQIRLQAEAFQARLKSWFEPLV EDMQRQWAGLVEKVQAAVGTSAAPVPSDNH |
| 4 | Hexamer ag 198 | LAGQPL |
| 5 | Hexamer ag 199 | AGQPLQ |
| 6 | Hexamer ag 200 | GQPLQE |
| 7 | Heptamer ag 198 | LAGQPLQ |
| 8 | Heptamer ag 199 | AGQPLQE |
| 9 | Octamer ag 198 | LAGQPLQE |
| 10-23 | HC CDRs | **Table 4** |
| 24-33 | LC CDRs | **Table 4** |
| 34-44 | VH and VL seqs | **Table 3** |
| 45 | Human ApoE4 | KVEQAVETEPEPELRQQTEWQSGQRWELALGRFWD YLRWVQTLSEQVQEELLSSQVTQELRALMDETMKEL KAYKSELEEQLTPVAEETRARLSKELQAAQARLGADM EDVRGRLVQYRGEVQAMLGQSTEELRVRLASHLRKL RKRLLRDADDLQKRLAVYQAGAREGAERGLSAIRERL GPLVEQGRVRAATVGSLAGQPLQERAQAWGERLRA RMEEMGSRTRDRLDEVKEQVAEVRAKLEEQAQQIRL QAEAFQARLKSWFEPLVEDMQRQWAGLVEKVQAAV GTSAAPVPSDNH |
| 46 | 158-233 of FL ApoE | RLAVYQAGAR EGAERGLSAIR ERLGPLVEQG RVRAATVGSL AGQPLQERAQ AWGERLRARM EEMGSRTRDR LDEVK |
| 47 | 200-233 of FL ApoE | GQPLQERAQA WGERLRARME EMGSRTRDRL DEVK |
| 48 | 200-299-10HIS | GQPLQERAQAWGERLRARMEEMGSRTRDRLDEVKE QVAEVRAKLEEQAQQIRLQAEAFQARLKSWFEPLVE DMQRQWAGLVEKVQAAVGTSAAPVPSDNHHHHHHH HHHH |
| 49 | 10HIS-ApoE4-FL | HHHHHHHHHKVEQAVETEPEPELRQQTEWQSGQR WELALGRFWDYLRWVQTLSEQVQEELLSSQVTQELR ALMDETMKELKAYKSELEEQLTPVAEETRARLSKELQ AAQARLGADMEDVRGRLVQYRGEVQAMLGQSTEEL RVRLASHLRKLRKRLLRDADDLQKRLAVYQAGAREG AERGLSAIRERLGPLVEQGRVRAATVGSLAGQPLQE RAQAWGERLRARMEEMGSRTRDRLDEVKEQVAEVR AKLEEQAQQIRLQAEAFQARLKSWFEPLVEDMQRQW AGLVEKVQAAVGTSAAPVPSDNH |
| 50 | 200G immunogen | GQPLQE-Acp-C |
| 51 | 199A immunogen | AGQPLQ-Acp-C |
| 52 | 198L immunogen | LAGQPL-Acp-C |
| 53 | Neg ctrl peptide | AATVGSLAGQPLQER-Acp-C |
| 54 | 198L new imm | LAGQPLQE-Acp-C |
| 55 | 199A new imm | AGQPLQE-Acp-C |

## Claims

1. An antibody or antigen binding portion thereof that binds to a fragment of apolipoprotein E (ApoE), wherein the fragment has
- an apparent molecular weight of 12 kDa as measured by SDS-PAGE, and
- an N-terminus corresponding to an amino acid in full-length apolipoprotein E which is selected from the group consisting of amino acids L198, A199 and G200; and wherein the antibody or antigen binding portion thereof binds to an epitope comprising the N-terminus of the fragment.

2. The antibody or antigen binding portion thereof according to claim 1, wherein the antibody or antigen binding portion thereof binds selectively to the ApoE fragment and does not bind to full-length apolipoprotein E.

3. The antibody or antigen binding portion thereof according to claim 1 or claim 2, wherein said fragment of apolipoprotein E is selected from
i) a fragment consisting of the amino acid sequence of any one of SEQ ID NOs:1-3; and
ii) a fragment having at least 80% identity to any one of SEQ ID NOs: 1-3.

4. The antibody or antigen binding portion thereof according to any one of claims 1-3, wherein the antibody or antigen binding portion thereof binds to an epitope comprising amino acid residues selected from the following:
(i) amino acid residues 200-205 in full-length ApoE (GQPLQE);
(ii) amino acid residues 199-204 in full-length ApoE (AGQPLQ);
(iii) amino acid residues 199-205 in full-length ApoE (AGQPLQE);
(iv) amino acid residues 198-203 in full-length ApoE (LAGQPL);
(v) amino acid residues 198-204 in full-length ApoE (LAGQPLQ); and
(vi) amino acid residues 198-205 in full-length ApoE (LAGQPLQE).

5. The antibody or antigen binding portion thereof according to any one of claims 1-4, wherein said fragment of apolipoprotein E consists of the amino acid sequence SEQ ID NO:1, optionally wherein the epitope comprises amino acid residues 200-205 in full-length apolipoprotein E.

6. A method of producing an antibody or an antigen binding portion thereof, comprising a step of immunizing a host mammal with a peptide immunogen comprising an N-terminal amino acid sequence selected from the group consisting of LAGQPL (SED ID NO:4), AGQPLQ (SEQ ID NO:5), GQPLQE (SEQ ID NO:6), LAGQPLQ (SEQ ID NO:7), AGQPLQE (SEQ ID NO:8) and LAGQPLQE (SEQ ID NO:9).

7. The method according to claim 6, wherein said N-terminal amino acid sequence is GQPLQE (SEQ ID NO:6).

8. An antibody or antigen binding portion thereof, obtainable by a method according to claim 6 or claim 7.

9. The antibody or antigen binding portion thereof according to any one of claims 1-5 or 8, wherein the antibody or antigen binding portion thereof comprises a variable heavy chain domain (VH) comprising three CDR sequences (CDR-H1, CDR-H2 and CDR-H3), wherein the three VH CDR sequences are independently selected from:
- CDR-H1 selected from the group consisting of SEQ ID NO: 10, 15, 18 and 21;
- CDR-H2 selected from the group consisting of SEQ ID NO: 11, 13, 16, 19 and 22; and
- CDR-H3 selected from the group consisting of SEQ ID NO: 12, 14, 17, 20 and 23.

10. The antibody or antigen binding portion thereof according to any one of claims 1-5 and 8-9, wherein the antibody or antigen binding portion thereof comprises a variable light chain domain (VL) comprising three CDR sequences (CDR-L1, CDR-L2 and CDR-L3), wherein the three VL CDR sequences are independently selected from:
- CDR-L1 selected from the group consisting of SEQ ID NO: 24, 27, 29, 31 and 32;
- CDR-L2 being SEQ ID NO: 25; and
- CDR-L3 selected from the group consisting of SEQ ID NO: 26, 28, 30 and 33.

11. The antibody or antigen binding portion thereof according to any one of claims 1-5 and 8-10, wherein the antibody or antigen binding portion thereof comprises a heavy chain variable domain (VH) comprising or consisting of an amino acid sequence selected from:
i) the group consisting of SEQ ID NOs: 34, 36, 38, 40, 42 and 43; and
ii) a sequence having at least 70 % identity to any one of SEQ ID NOs: 34, 36, 38, 40, 42 and 43.

12. The antibody or antigen binding portion thereof according to any one of claims 1-5 and 8-11, wherein the antibody or antigen binding portion thereof comprises a light chain variable domain (VL) comprising or consisting of an amino acid sequence selected from:
i) the group consisting of SEQ ID NOs: 35, 37, 39, 41 and 44; and
ii) a sequence having at least 70 % identity to any one of SEQ ID NO:35, 37, 39, 41 and 44.

13. A pharmaceutical composition comprising an antibody or antigen binding portion thereof according to any one of claims 1-5 and 8-12 and at least one pharmaceutically acceptable excipient or carrier.

14. An antibody or antigen binding portion thereof according to any one of claims 1-5 and 8-12, or pharmaceutical composition according to claim 13, for use in therapy.

15. A method of detecting or diagnosing a neurological disorder or a disorder **characterized by** a loss of cognitive memory capacity in a subject, the method comprising contacting a sample obtained from the subject with an antibody or antigen binding portion thereof according to any one of claims 1-5 and 8-12.
